# EUROPEAN PATENT APPLICATION

(11) **EP 4 692 172 A1**
(43) Date of publication of application: **11.02.2026**
(21) Application number: 24779389.6
(22) Date of filing: 12.03.2024
(51) Int. Cl.: C08G 77/50, A61K 8/894, A61Q 1/02, A61Q 1/04, A61Q 17/04, A61Q 19/00, C08G 77/46, C08L 83/12, C08L 83/14, C09K 23/54

(54) **ORGANOPOLYSILOXANE, DISPERSION, AND COSMETIC PRODUCT**

(30) Priority: 27.03.2023 JP 2023049920
(71) Applicant: SHIN-ETSU CHEMICAL CO., LTD., Tokyo 1000005 (JP)
(72) Inventor: ABE, Takuya, Annaka-shi, Gunma 379-0224 (JP); KONISHI, Masayuki, Tokyo 100-0005 (JP)
(74) Representative: Mewburn Ellis LLP
(86) International application number: PCT/JP2024/009458
(87) International publication number: WO 2024/203259

(57) **Abstract**

An organopolysiloxane represented by formula (1) and having excellent dispersibility of powders. 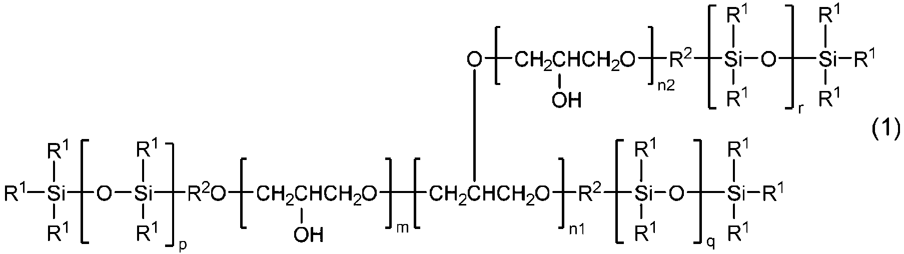 [In the formula, R¹ is independently a group selected from an alkyl group having 1-20 carbon atoms, an aryl group having 6-20 carbon atoms, and an aralkyl group having 7-20 carbon atoms. R² is independently a C3-15 divalent organic group which may have an intervening oxygen atom. p is 0 ≤ p ≤ 9, q is 0 ≤ q ≤ 200, r is 0 ≤ r ≤ 200, m is 0 ≤ m ≤ 10, and n1 is 0 ≤ n ≤ 15 (note that when m = 0 or 1, 1 ≤ n1 ≤ 5 is satisfied), n2 is an integer where 0 ≤ n2 ≤ 5. Note bonding of that each glycerol unit identified by m and n1 may block or random. ]

## Description

### TECHNICAL FIELD

The present invention relates to an organopolysiloxane, a dispersion containing the same, and cosmetic compositions including any of these. In the present invention, compositions for cosmetic compositions are also written as cosmetic compositions.

### BACKGROUND ART

Silicone surfactants are conventionally used frequently as emulsifiers for emulsifying silicones. In particular, polyglycerin-modified silicones show adhesiveness to the skin that is offered by the polyglycerin and have a light feel that is characteristic of silicones, thus finding wide use in emulsified cosmetic compositions and the like. However, it is known that the use of such silicones tends to cause sensory stickiness (Patent Document 1).

Meanwhile, metal oxide powders, such as titanium oxide and zinc oxide, that are generally used in sunscreen cosmetic compositions are in the form of so-called microparticulate metal oxide powders with an average particle size of 100 nm or less in order to attain enhanced transparency and higher UV-shielding effects. However, metal oxide particles having such a small size bond strongly to one another due to the increased surface area and consequently tend to be aggregated easily. It is therefore important that these microparticulate metal oxide powders, when used in sunscreen cosmetic compositions, can be contained stably without being aggregated.

Patent Document 2 proposes a silicone-branched polyglycerin-modified silicone that gives high dispersibility to powders. However, this structure comes with high viscosity. Patent Documents 3 and 4 propose ABA copolymers as powder dispersibility stabilizers. However, the silicone chain lengths and how the bias in silicone chain lengths will affect the dispersibility are not sufficiently studied.

### PRIOR ART DOCUMENTS

### PATENT DOCUMENTS

Patent Document 1: JP-A 2002-3334
Patent Document 2: WO 2016/178380
Patent Document 3: JP-A 2006-218472
Patent Document 4: JP-A 2012-207078

### SUMMARY OF INVENTION

### TECHNICAL PROBLEM

The present invention has been made in view of the circumstances discussed above. It is therefore an object of the present invention to provide an organopolysiloxane that allows a powder to exhibit excellent dispersibility. Another object is to provide a dispersion with good stability, and a cosmetic composition with excellent feeling on use and spreadability.

### SOLUTION TO PROBLEM

The present inventors carried out extensive studies directed to achieving the above objects and have consequently found that an organopolysiloxane in which each end of a polyglycerin is modified with an organopolysiloxane chain having a specific chain length allows a powder to exhibit excellent dispersibility. Based on the fact that the above organopolysiloxane offers high dispersion stability of powder, the present inventors have further developed a highly stable dispersion containing the organopolysiloxane, and a cosmetic composition with excellent feeling on use and spreadability that includes the organopolysiloxane or the dispersion. The present invention has been thus completed.

Thus, the present invention provides the following inventions:
1. An organopolysiloxane represented by formula (1) below: 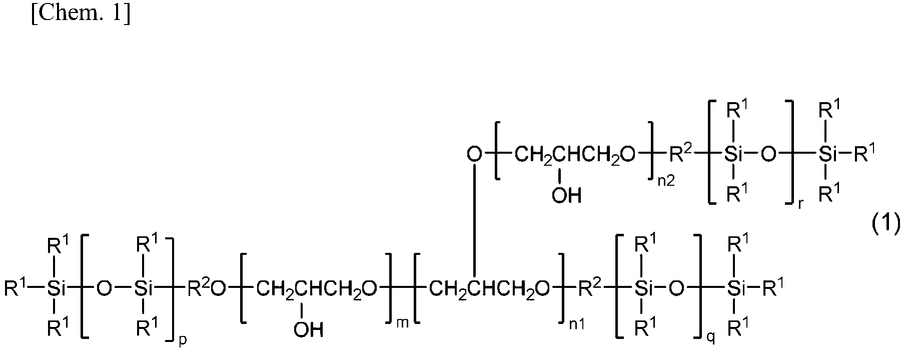 [wherein R¹ independently at each occurrence is a group selected from C1-C20 alkyl groups, C6-C20 aryl groups, and C7-C20 aralkyl groups; R² independently at each occurrence is a C3-C15 divalent organic group optionally interrupted by an oxygen atom; p is an integer of 0 ≤ p ≤ 9; q is an integer of 0 ≤ q ≤ 200; r is an integer of 0 ≤ r ≤ 200; m is an integer of 0 ≤ m ≤ 10; n1 is an integer of 0 ≤ n1 ≤ 5 (with a proviso that when m = 0 or 1, 1 ≤ n1 ≤ 5); n2 is an integer of 0 ≤ n2 ≤ 5; and the glycerin units in the brackets followed by m and n1 are each optionally bonded to one another blockwise or randomly].
2. The organopolysiloxane according to 1, wherein in formula (1), n1 = 0 and m is 0 < m ≤ 10.
3. The organopolysiloxane according to 2, wherein in formula (1), p ≠ q.
4. The organopolysiloxane according to any one of 1 to 3, wherein the organopolysiloxane has a polystyrene-equivalent weight average molecular weight of 1,000 to 20,000.
5. The organopolysiloxane according to any one of 1 to 4, wherein in formula (1), q is 0 ≤ q ≤ 9 and r is 0 ≤ r ≤ 9.
6. The organopolysiloxane according to any one of 1 to 4, wherein in formula (1), q is 10 ≤ q ≤ 200.
7. A cosmetic composition including the organopolysiloxane described in any one of 1 to 6.
8. A dispersion including the organopolysiloxane described in any one of 1 to 6, a powder, and an oil.
9. The dispersion according to 8, wherein the organopolysiloxane is represented by formula (1) in which q is 0 ≤ q ≤ 9 and r is 0 ≤ r ≤ 9, and the powder has an average primary particle size of less than 15 nm.
10. The dispersion according to 8, wherein the organopolysiloxane is represented by formula (1) in which 10 ≤ q ≤ 200, and the powder has an average primary particle size of 15 nm or more.
11. A cosmetic composition including the dispersion described in any one of 8 to 10.

### ADVANTAGEOUS EFFECTS OF INVENTION

The organopolysiloxane provided according to the present invention allows a powder to exhibit excellent dispersion stability. Treating a powder with the organopolysiloxane can provide a dispersion having good stability. Furthermore, the organopolysiloxane or a dispersion using the organopolysiloxane is useful in a cosmetic composition and the resultant cosmetic composition exhibits a good feeling on use and excellent spreadability.

### DESCRIPTION OF EMBODIMENTS

The present invention will be described in detail below without limiting the scope of the present invention to the following description. The following description of the present invention focuses on the use in cosmetic compositions, but the application is not particularly limited thereto. In the present invention, the name of an ingredient is written as the name cited in *Kesho̅hin Hyo̅ji Meisho̅* [Japanese Cosmetic Labeling Names] or in the International Nomenclature of Cosmetic Ingredients (INCI). When the names from *Kesho̅hin Hyo̅ji Meisho̅* and the INCI are the same, the name from *Kesho̅hin Hyo̅ji Meisho̅* or the INCI name is sometimes omitted.

### [Organopolysiloxanes]

An organopolysiloxane of the present invention is represented by formula (1) below. Such organopolysiloxanes may be used singly, or two or more may be used in combination. 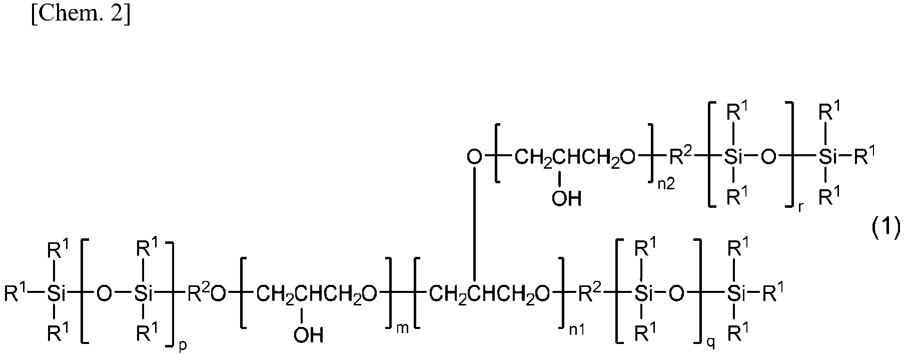 [In the formula, R¹ independently at each occurrence is a group selected from C1-C20 alkyl groups, C6-C20 aryl groups, and C7-C20 aralkyl groups; R² independently at each occurrence is a C3-C15 divalent organic group optionally interrupted by an oxygen atom; p is an integer of 0 ≤ p ≤ 9; q is an integer of 0 ≤ q ≤ 200; r is an integer of 0 ≤ r ≤ 200; m is an integer of 0 ≤ m ≤ 10; n1 is an integer of 0 ≤ n1 ≤ 5 (with the proviso that when m = 0 or 1, 1 ≤ n1 ≤ 5); n2 is an integer of 0 ≤ n2 ≤ 5; and the glycerin units in the brackets followed by m and n1 are each optionally bonded to one another blockwise or randomly.]

R¹ independently at each occurrence is a group selected from C1-C20 alkyl groups, C6-C20 aryl groups, and C7-C20 aralkyl groups. R¹ is preferably a C1-C15 alkyl group, a C6-C10 aryl group, a C7-C10 aralkyl group, or an alkyl group resulting from the substitution of the above alkyl group with a fluorine atom in place of part of the hydrogen atoms. Specific examples include methyl group, ethyl group, propyl group, butyl group, pentyl group, octyl group, decyl group, dodecyl group, cyclopentyl group, cyclohexyl group, phenyl group, tolyl group, and trifluoropropyl group. In particular, C1-C5 alkyl groups, phenyl group, and trifluoropropyl group are preferable.

R² independently at each occurrence is a C3-C15 divalent organic group optionally interrupted by an oxygen atom. In the above formula, R² independently at each occurrence is a C3-C15 divalent organic group optionally having an oxygen atom between any atoms in the group. Examples include -(CH₂)₃-, -(CH₂)₄-, -CH₂CH(CH₃)CH₂-, -(CH₂)₈-, -(CH₂)₁₁-, -(CH₂)₃-O-(CH₂)₂-, and-(CH₂)₂-O-(CH₂)₃-. Among these, -(CH₂)₃- and -CH₂CH(CH₃)CH₂-are preferable.

In the above formula, p is 0 ≤ p ≤ 9, preferably 1 ≤ p ≤ 9, and more preferably 3 ≤ p ≤ 9. If p is greater than the upper limit, the organopolysiloxane used as a dispersant does not adsorb well to a powder and causes poor dispersibility. The letter q is 0 ≤ q ≤ 200. When the organopolysiloxane is blended with a powder described later and when the average primary particle size of the powder is less than 15 nm, it is preferable that 0 ≤ q ≤ 9. When q is 9 or less, the organopolysiloxane adsorbs well to a powder and offers good dispersibility. When the average primary particle size of the powder is 15 nm or more, it is preferable that 10 ≤ q ≤ 200. When q is 10 or greater, the hydrophobic group is sufficiently long and particle aggregation is unlikely to occur, that is, good dispersibility is obtained. The letter r is 0 ≤ r ≤ 200. When the average primary particle size of the powder is less than 15 nm, it is preferable that 0 ≤ r ≤ 9. When r is 9 or less, the organopolysiloxane adsorbs well to a powder and offers good dispersibility. The average primary particle size of a powder is the average of randomly selected 100 particles measured with a transmission electron microscope (the same applies hereinbelow).

In the above formula, m is 0 ≤ m ≤ 10, preferably 1 ≤ m ≤ 8, and more preferably 1 < m ≤ 5. If m is greater than 10, the organopolysiloxane used as a dispersant exhibits so high a polarity that the compatibility between the dispersant and an oil, in particular, a silicone oil, may be deteriorated. Furthermore, the viscosity of the dispersant itself is increased, and the dispersant is poorly compatible with a powder and may cause low dispersibility. Furthermore, n1 is 0 ≤ n1 ≤ 5, preferably 0 ≤ n1 ≤ 3, more preferably 0 ≤ n1 ≤ 1, and still more preferably 0. When n1 = 0, m is 0 < m ≤ 10. When n1 is not 0, it is preferable that n1 = 1. When m = 0 or 1, 1 ≤ n1 ≤ 5, preferably 1 ≤ n1 ≤ 3, and more preferably n1 = 1. When n1 is 5 or less, the polarity of the dispersant is low and the compatibility between the dispersant and an oil is effectively increased. n2 is 0 ≤ n2 ≤ 5, preferably 1 ≤ n2 ≤ 3, and more preferably 1 ≤ n2 ≤ 2. If n2 is greater than 5, the dispersant exhibits so high a polarity that the compatibility between the dispersant and an oil, in particular, a silicone oil, may be deteriorated. The glycerin units in the brackets followed by m and n1 are each optionally bonded to one another blockwise or randomly. The siloxane units may be bonded in any order without limitation.

When the above formula is such that q is 0 ≤ q ≤ 9 and r is 0 ≤ r ≤ 9, the siloxane units are short and the organopolysiloxane adsorbs well to a powder having an average primary particle size of less than 15 nm and allows the powder to exhibit high dispersion stability. Thus, such an organopolysiloxane may be suitably used as a dispersant for a powder with a small average primary particle size.

When the above formula is such that q is 10 ≤ q ≤ 200, the siloxane units are long and the organopolysiloxane adsorbs well to a powder having an average primary particle size of 15 nm or more and allows the powder to exhibit high dispersion stability. Thus, such an organopolysiloxane may be suitably used as a dispersant for a powder with a large average primary particle size. As can be understood from the above, the organopolysiloxane of the present invention, by being optimized in structure, may be suitably used as a dispersant capable of allowing a powder of any particle size to be favorably dispersed.

When the above formula is such that n1 = 0 and m is 0 < m ≤ 10, the organopolysiloxane as a dispersant exhibits a low viscosity and can uniformly treat a powder. Thus, such an organopolysiloxane may be suitably used as a dispersant. When p ≠ q, the organopolysiloxane performs well in adsorbing to a powder and also in compatibility with an oil, thus allowing for higher dispersion stability of the powder.

### [Methods for synthesizing the organopolysiloxanes]

The organopolysiloxane of the present invention may be synthesized by any method without limitation. The organopolysiloxane may be obtained by the addition reaction of a polyglycerin compound having two or more alkenyl groups as a raw material, with one, or two or more organohydrogenpolysiloxanes having a hydrosilyl group at one end.

Specifically, the organopolysiloxane may be obtained by the hydrosilylation reaction of a polyglycerin compound having two or more alkenyl groups, represented by formula (2) below, with one, or two or more organohydrogenpolysiloxanes having a hydrosilyl group at one end, represented by formula (3) below. The hydrosilylation reaction may be performed in the presence of a platinum catalyst or a rhodium catalyst. 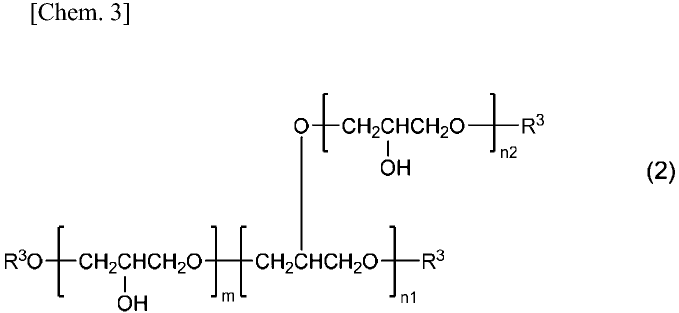 [In the formula, R³ independently at each occurrence is a C3-C15 alkenyl group optionally having an oxygen atom between any atoms in the group; m is an integer of 0 ≤ m ≤ 10; n1 is an integer of 0 ≤ n1 ≤ 5 (with the proviso that when m = 0 or 1, 1 ≤ n1 ≤ 5); n2 is an integer of 0 ≤ n2 ≤ 5; and the glycerin units in the brackets followed by m and n1 are each optionally bonded to one another blockwise or randomly.] 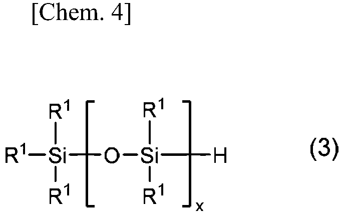 [In the formula, R¹ independently at each occurrence is a group selected from C1-C20 alkyl groups, C6-C20 aryl groups, and C7-C20 aralkyl groups; and x is p, q, or r described hereinabove.]

The polyglycerin compound having two or more alkenyl groups may be obtained by subjecting an epoxy compound, such as monoallyl glycidyl ether, monooctenyl glycidyl ether, glycidol, or glycidyl methyl ether, to epoxy ring-opening reaction with a hydroxyl-containing compound, such as glycerin, diglycerin, glycerin monoallyl ether, or glycidol, in the presence of an alkali catalyst.

The ring-opening reaction between a hydroxyl group and an epoxy group is known. The type of alkali catalyst is not particularly limited and use may be made of, for example, alkali metal hydroxides, such as potassium hydroxide and sodium hydroxide; alkali metal hydrogencarbonates, such as sodium hydrogencarbonate and potassium hydrogencarbonate; alkali metal carbonates, such as sodium carbonate and potassium carbonate; and sodium methoxide. The amount in which the alkali catalyst is added is 0.2 to 2 mol%, preferably 0.2 to 1 mol%, based on 100 mol% of the hydroxyl-containing compound. The product from the ring-opening reaction between the hydroxyl group and the epoxy group includes various isomers depending on the proportions of the raw materials and is usually a mixture of such isomers.

For example, the reaction of 1 mole of glycerin with 2 moles of allyl glycidyl ether theoretically gives 14 types of structural isomers illustrated below:

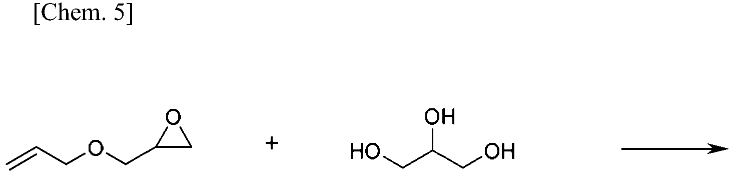

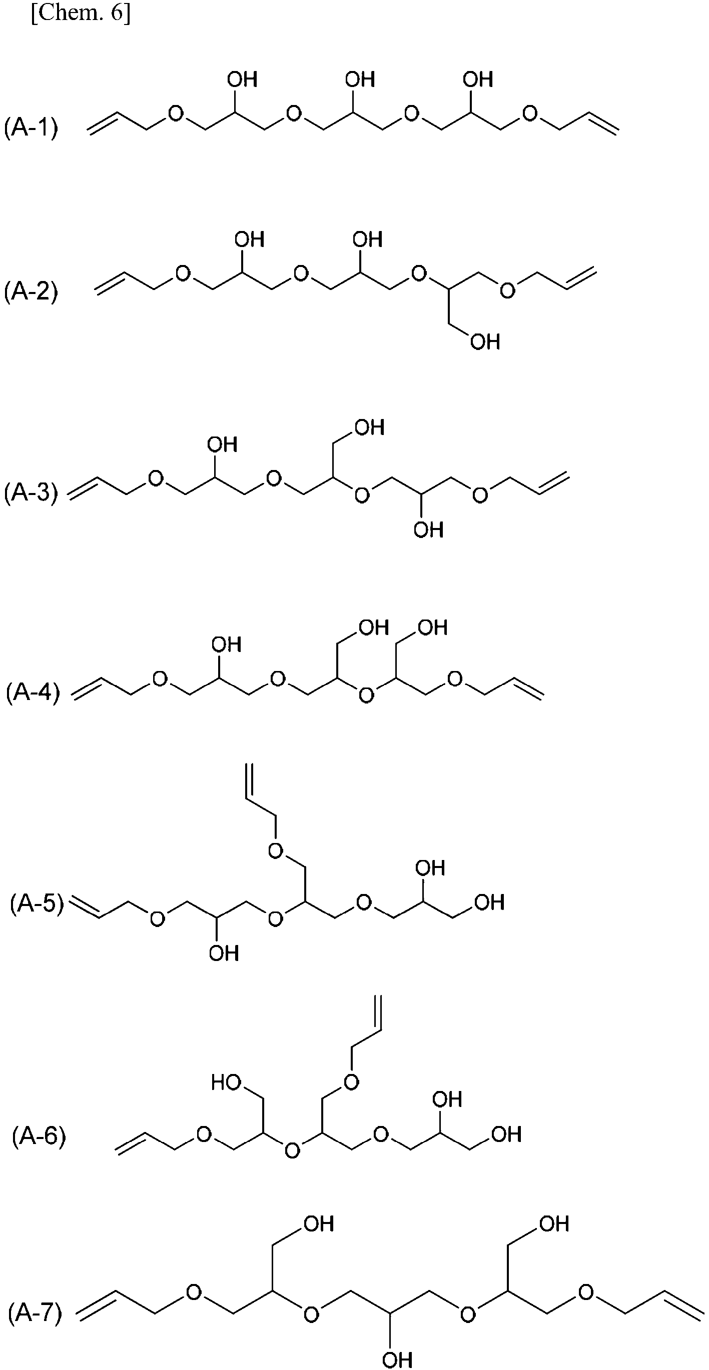

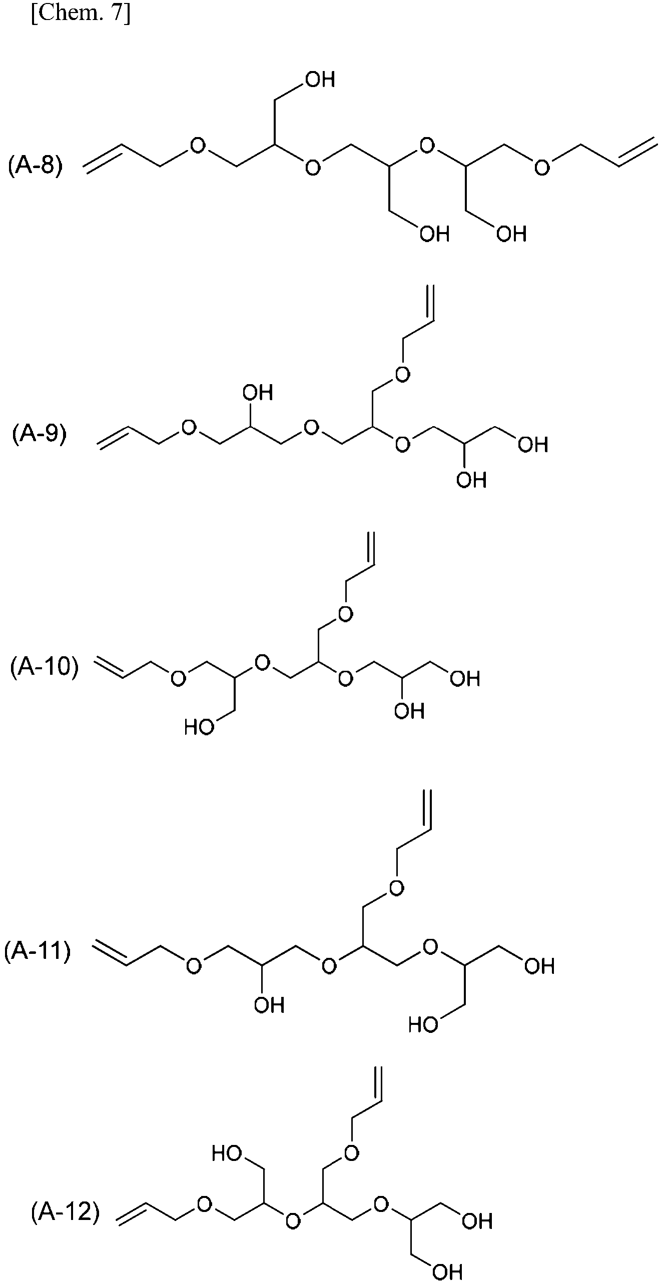

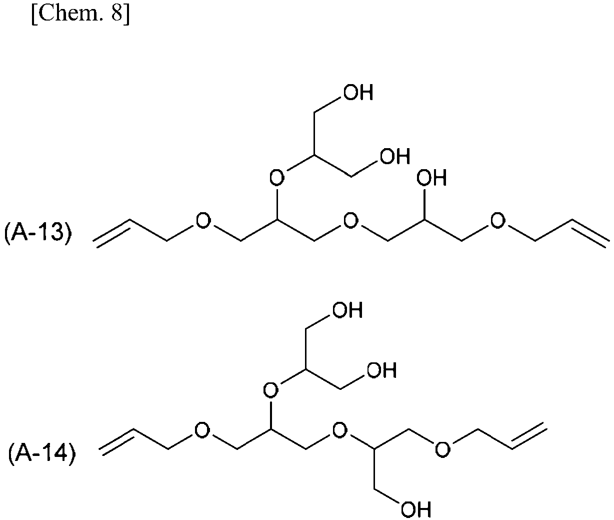

The production method by the hydrosilylation reaction will be described in greater detail below. In the step of hydrosilylation reaction between the polyglycerin compound having two or more alkenyl groups, represented by formula (2) above, with the organohydrogenpolysiloxane having a hydrosilyl group at one end, represented by formula (3) above, the hydrosilyl/alkenyl molar ratio is preferably 0.5 to 2.0, and more preferably 0.8 to 1.2.

This hydrosilylation reaction is preferably performed in the presence of a platinum catalyst or a rhodium catalyst. Examples of preferred catalysts include chloroplatinic acid, alcohol-modified chloroplatinic acids, and chloroplatinic acid-vinyl siloxane complexes. In view of the fact that the reaction in the presence of an excessively large amount of the catalyst produces a colored product, the amount in terms of platinum or rhodium is preferably 50 ppm (by mass) or less, and more preferably 20 ppm or less.

Where necessary, the above addition reaction may be carried out in the presence of an organic solvent. Examples of the organic solvents include cyclic organopolysiloxanes, such as octamethylcyclotetrasiloxane, decamethylcyclopentasiloxane, and dodecamethylcyclohexasiloxane; aromatic hydrocarbons, such as toluene and xylene; ketone organic solvents, such as acetone, methyl ethyl ketone, diethyl ketone, and methyl isobutyl ketone; aliphatic hydrocarbons, such as hexane, heptane, octane, and cyclohexane; monohydric aliphatic alcohols, such as methanol, ethanol, 1-propanol, 2-propanol, 1-butanol, 2-methyl-1-propanol, 2-butanol, 2-methyl-2-propanol, 1-pentanol, 2-methylbutanol, 2-pentanol, 1-hexanol, 2-methylpentanol, 1-heptanol, 1-octanol, 1-nonanol, and 1-decanol; and dihydric aliphatic alcohols, such as ethylene glycol and 1,2-propylene glycol. Among these, ethanol, 1-propanol, and 2-propanol are preferable from the point of view of reactivity.

The amount in which the solvent is used is preferably 1 to 80 mass%, and more preferably 5 to 50 mass% of the whole reaction liquid (system). When the amount is in the above range, the reaction system is kept uniform and the reaction proceeds efficiently.

While the addition reaction conditions are not particularly limited, the reaction is preferably performed by heating under reflux at a temperature of 50 to 150°C, more preferably 80 to 120°C, for about 1 to 10 hours.

The method may include, after the addition reaction, a step of removing the spent rhodium catalyst or platinum catalyst with activated carbon. The amount in which the activated carbon is used is preferably 0.001 to 5.0 mass%, in particular, 0.01 to 1.0 mass%, of the whole system. This amount ensures that coloring of the reaction product will be less.

The organopolysiloxane resulting from the addition reaction may contain an unreacted hydrosilyl group. When the organic solvent used in the addition reaction is an aliphatic alcohol, alcohol exchange reaction and dehydrogenation reaction occur as side reactions. In such a case, the product may contain a residual alkoxy group.

Where necessary, the method may include, after the addition reaction, a step of substituting residual hydrosilyl groups with hydroxysilyl groups. The hydrosilyl groups may be inactivated over time by dehydrogenation reaction, which may result in the generation of hydrogen gas. This is problematic from the point of view of safety. Thus, the addition of a step of substituting hydrosilyl groups with hydroxysilyl groups is preferable particularly when the organopolysiloxane will be used in such an application as cosmetic compositions.

The step of substituting hydrosilyl groups with hydroxysilyl groups may be performed by hydrolyzing the unreacted hydrosilyl groups in the presence of a basic catalyst, such as an alkali metal carbonate, an alkali metal hydrogencarbonate, or an alkali metal hydroxide, and then adding an acid catalyst in an amount equal to the molar equivalent of the basic catalyst to neutralize the system. Specific examples of the basic catalysts include strong basic catalysts, such as lithium hydroxide, sodium hydroxide, potassium hydroxide, calcium hydroxide, and barium hydroxide, and weak basic catalysts, such as sodium carbonate, calcium carbonate, and sodium hydrogencarbonate. To promote the dehydrogenation reaction, it is particularly preferable to use a strong basic catalyst, specifically, sodium hydroxide. Specific examples of the acid catalysts include inorganic acids, such as hydrochloric acid, sulfuric acid, sulfurous acid, fuming sulfuric acid, and phosphoric acid; sulfonic acids, such as p-toluenesulfonic acid, methanesulfonic acid, and trifluoromethanesulfonic acid; and carboxylic acids, such as formic acid, acetic acid, propionic acid, trifluoroacetic acid, benzoic acid, citric acid, and oxalic acid. It is generally preferable to use the acid and the base in combination with water and perform heating at a temperature below the boiling point of water rather than using the acid and the base alone. Through this step, the hydrosilyl groups (SiH groups) are substituted with hydroxysilyl groups (SiOH groups).

After the addition reaction, a deodorizing step of reducing odor may be performed as required. The organopolysiloxanes develop an odor over time. Thus, the addition of a deodorizing step is preferable particularly when the organopolysiloxane will be used in such an application as cosmetic compositions. The odor-developing mechanism of general polyether-modified silicones may be probably explained as follows. When addition reaction is performed between an allyl-etherified polyether and a hydrogen polyorganosiloxane in the presence of a platinum catalyst, the allyl group undergoes internal transfer as side reaction to give propenyl-etherified polyether. This propenyl-etherified polyether has no addition reactivity with hydrogen polyorganosiloxanes and thus remains as an impurity in the system. Water acts on this propenyl-etherified polyether to hydrolyze the propenyl ether, giving rise to bad odor-causing propionaldehyde. In addition, it is known that the above hydrolysis reaction is further promoted in the presence of an acid catalyst. When a polyether-modified silicone is used in an aqueous cosmetic composition, the liquid becomes acidic over time due to the oxidative deterioration of the polyether. This promotes the hydrolysis reaction described above and causes odor.

For example, the deodorizing step may be typically performed in two ways. In the first case, an acid catalyst is added to the solution after the addition reaction to hydrolyze completely the propenyl ether remaining in the system, and the resulting propionaldehyde is distilled away.

Specific examples of the acid catalysts used in the first case include inorganic acids, such as hydrochloric acid, sulfuric acid, sulfurous acid, fuming sulfuric acid, and phosphoric acid; sulfonic acids, such as p-toluenesulfonic acid, methanesulfonic acid, and trifluoromethanesulfonic acid; and carboxylic acids, such as formic acid, acetic acid, propionic acid, trifluoroacetic acid, benzoic acid, oxalic acid, and citric acid. These acids are used in combination with water. When the acid should be removed after being used, it is preferable to use an acid with a low boiling point, such as hydrochloric acid, formic acid, acetic acid, or trifluoroacetic acid. While the use of a strong acid, such as hydrochloric acid or trifluoroacetic acid, is preferable from the point of view of treatment efficiency, a cyclic organopolysiloxane used as a solvent may be ring-opened in the presence of a strong acid and is therefore preferably used together with a weak acid, such as citric acid or acetic acid.

The treatment temperature is preferably 80°C or below to prevent the oxidation of hydrophilic groups. The amount in which the acidic aqueous solution is added is preferably 0.1 to 100 mass%, and more preferably 5 to 30 mass% relative to the organic group-modified organosilicon resin.

From the point of view of productivity, the step is preferably performed in such a manner that the aqueous solution is added to the solution after the reaction so that the pH will be 7 or less, the mixture being then heated and stirred, and the product is recovered by stripping purification. The stripping purification may be performed at room temperature or under reduced pressure. The temperature condition is preferably 120°C or below. In order to obtain efficiency under this temperature condition, it is preferable to perform the stripping purification under reduced pressure or to perform the purification at normal pressure under the flow of an inert gas, such as nitrogen or argon.

In the second case, hydrogen is added to the solution after the addition reaction to alkylate unsaturated double bonds (so-called hydrogenation reaction), and thereby the over-time occurrence of aldehyde compounds is controlled stably.

The hydrogenation reaction may be carried out using hydrogen or a metal hydride and the reaction may be homogeneous or heterogeneous. These options may be adopted singly or may be combined. Heterogeneous catalytic hydrogenation reaction using a solid catalyst is preferable in view of the advantageous fact that the catalyst used does not remain in the product.

Examples of the solid catalysts include nickel, palladium, platinum, rhodium, cobalt, chromium, copper, iron, and compounds thereof. In this case, the reaction may not involve a catalyst carrier or may use, for example, activated carbon, silica, silica alumina, alumina, or zeolite. These catalysts may be used singly or may be combined. Raney nickel, which is economically advantageous, is most preferable. Raney nickel is usually used after being developed with an alkali. It is therefore particularly necessary to carefully measure the pH of the reaction solution. Because the use of Raney nickel makes the reaction system weakly alkaline, the hydrolysis reaction using an acidic aqueous solution is particularly effective for deodorization.

In general, the hydrogenation reaction is preferably performed at a pressure of 1 to 100 MPa and a temperature of 50 to 200°C. The hydrogenation reaction may be batchwise or continuous. In the case of batchwise reaction, the reaction time depends on factors, such as the amount of the catalyst and the temperature, but is generally 3 to 12 hours. The hydrogen pressure may be appropriately controlled to a fixed pressure. The endpoint of the hydrogenation reaction is the point where a constant hydrogen pressure is reached and may be determined by carefully observing the pressure change.

The amount of aldehyde contained in the crosslinked organosilicon resin purified by the above acid treatment or hydrogenation reaction treatment may be preferably 70 ppm or less, more preferably 20 ppm or less, and still more preferably 10 ppm or less.

The two types of deodorizing step described above may be combined. The acid treatment process can remove aldehyde compounds through decomposition, but has a limit in removing all the unsaturated double bonds and is consequently incapable of completely suppressing the occurrence of odor-causing aldehydes from unsaturated double bonds. The hydrogenation reaction process can eliminate unsaturated double bonds and thereby can reduce the amount of aldehyde compounds that are generated. However, aldehyde condensates formed by condensation of part of the aldehydes remain in the system even after the above treatment and are difficult to remove even by stripping purification. Thus, perfect deodorization is possible by performing hydrogenation reaction on the solution after the addition reaction to alkylate the remaining unsaturated double bonds, and subsequently adding an acid catalyst to decompose the aldehyde condensates in the system.

### [Properties of the organopolysiloxanes]

The weight average molecular weight of the organopolysiloxane of the present invention is preferably 1,000 to 20,000, more preferably 1,000 to 10,000, and still more preferably 1,000 to 7,000.

When the weight average molecular weight is 1,000 or more, the organopolysiloxane is more effective in suppressing the aggregation of particles when used as a dispersant. When the weight average molecular weight is 20,000 or less, the adsorptivity to a powder is enhanced. The above range is also preferable from the points of view of performance and workability, such as filtration. For these reasons, the molecular weight of the organopolysiloxane of the present invention is preferably 1,000 to 20,000.

The weight average molecular weight in the present invention is determined as the polystyrene-equivalent weight average molecular weight by gel permeation chromatography (GPC) analysis under the following conditions:

### [Measurement conditions]

| | |
|---|---|
| Developing solvent: | tetrahydrofuran (THF) |
| Flow rate: | 0.6 mL/min |
| Detector: | differential refractive index detector (RI) |
| Columns: | TSK Guardcolumn Super H-H |
| | TSKgel SuperHM-N (6.0 mm I.D. × 15 cm × 1) |
| | TSKgel SuperH2500 (6.0 mm I.D. × 15 cm × 1) (all manufactured by TOSOH CORPORATION) |
| Column temperature: | 40°C |
| Sample injection volume: | 50 µL (0.3 mass% THF solution) |

### [Dispersions]

The organopolysiloxane of the present invention allows a powder to attain excellent dispersion stability and therefore can provide a dispersion having excellent stability, for example, stability in powder dispersibility, viscosity, and hardness. For example, the dispersion may include the organopolysiloxane of the present invention, an oil, and a powder. When the organopolysiloxane of the present invention is used in a dispersion, the amount thereof is determined appropriately and is preferably 0.1 to 30 mass%, more preferably 0.5 to 15 mass%, and still more preferably 1 to 10 mass% in the dispersion including the organopolysiloxane of the present invention, an oil, and a powder. The viscosity of the liquid dispersion at 25°C is not particularly limited. When the production is made with a medium agitating mill, such as a bead mill, the viscosity is preferably less than 1,000 mPa·s in view of the ease of filtering the beads and the ease of blending into cosmetic compositions. From the point of view of feeling on use, the viscosity is more preferably less than 750 mPa·s, and still more preferably less than 500 mPa·s. The lower limit is not particularly limited, but the concentration variation in the dispersion tends to be small when the viscosity is 100 mPa·s or more. The dispersion is analyzed using a Brookfield viscometer with, for example, Spindle LV-2 at 30 rotations, and the viscosity is measured after 60 seconds. When the dispersion is a paste, the hardness is not particularly limited. When the production is made with a three-roll mill, the hardness is preferably 100 or less to facilitate the processing. The hardness is more preferably less than 80, and still more preferably less than 50 to facilitate blending in cosmetic compositions. The lower limit is not particularly limited, but the concentration variation in the dispersion tends to be small when the hardness is 5 or more. The hardness is a value measured with a rheometer, for example, Rheometer RT-2002D·D (manufactured by RHEOTECH, probe: 10 mmφ, penetration depth: 10 mm, sample stage rising speed: 5 cm/min, temperature: 25°C, range: 200).

### • Powders

When the organopolysiloxane of the present invention is used in the dispersion, the powder that is dispersed is not particularly limited and may be any raw material usually added to cosmetic compositions. Examples thereof include microparticulate metal oxide powders, hydrophobized coloring pigments, inorganic powders, metal powders, organic powders, and inorganic-organic composite powders. These powders are described in detail below.

### • Microparticulate metal oxide powders

The microparticulate metal oxide used in the present invention is one, or two or more selected from microparticulate titanium oxide (labeling name, INCI: Titanium Dioxide), iron-containing titanium oxide, zinc oxide (labeling name, INCI: Zinc Oxide), cerium oxide (labeling name, INCI: Cerium Oxide), and composites thereof. A composite powder of the above metal oxide with other powder may also be used. The average primary particle size is preferably 200 nm or less, and more preferably 120 nm or less. If the particle size is larger than the above, the UV-protecting function is lowered and the powder applied leaves a white cast on the skin.

The microparticulate metal oxide may be untreated or may have undergone well-known surface treatment generally used for cosmetics. For example, inorganic treatments include coating with silica (labeling name, INCI: Silica), coating with alumina (labeling name, INCI: Alumina), and coating with Al hydroxide (labeling name, INCI: Aluminum Hydroxide); and organic treatments include silanes or silylating agents, such as triethoxycaprylylsilane (labeling name, INCI: Triethoxycaprylylsilane), silicone oils, such as dimethicone (labeling name, INCI: Dimethicone), hydrogen dimethicone (labeling name, INCI: Hydrogen Dimethicone), and triethoxysilylethyl polydimethylsiloxyethyl hexyl dimethicone (labeling name, INCI: Triethoxysilylethyl Polydimethylsiloxyethyl Hexyl Dimethicone), waxes, paraffins, organic fluorine compounds, such as perfluoroalkyl phosphate salts, surfactants, amino acids, such as N-acylglutamic acid, and metal soaps, such as Al stearate (labeling name, INCI: Aluminum Stearate) and Mg myristate (labeling name, INCI: Magnesium Myristate). In particular, triethoxysilylethyl polydimethylsiloxyethyl hexyl dimethicone (labeling name, INCI: Triethoxysilylethyl Polydimethylsiloxyethyl Hexyl Dimethicone) exhibits high dispersibility to both silicones and oils, and is therefore applied to sunscreens and foundations. Treatment with triethoxycaprylylsilane (labeling name, INCI: Triethoxycaprylylsilane) and metal soaps is preferable in terms of the compatibility with ingredients (B) and ingredients (D) described later. These surface treatments may be applied singly, or two or more may be combined depending on the purpose.

Microparticulate metal oxides that are surface-treated as described above are available in the market. For example, microparticulate titanium oxide is commercially available under the trade names of STR-100C-LP, STR-100A-LP, STR-100W, STR-100W-LP, STR-100C-LF, and STR-40-LP (manufactured by SAKAI CHEMICAL INDUSTRY CO., LTD.), MT-N1, MT-01, MT-05, MT-100Z, MT-100TV, MT-100AQ, MT-100WP, MTY-100SAS, MT-150EX, MT-500B, MT-505SAS, MT-700B, MT-700Z, MT-014Z, and SMT-500SAS (manufactured by TAYCA Co., Ltd.), and ST-455, ST-455WS, ST-457ECS, and ST-495M (manufactured by Titan Kogyo, Ltd.). Microparticulate zinc oxide is commercially available under the trade names of FINEX-50S-LP2, FINEX-30S-LP2, FINEX-50W, FINEX-30W, FINEX-50W-LP2, FINEX-52W-LP2, FINEX-30W-LP2, FINEX-33W-LP2, FINEX-50-LPT, FINEX-25-LPT, FINEX-50S-LPT, and FINEX-30S-LPT (manufactured by SAKAI CHEMICAL INDUSTRY CO., LTD.), and MZ-150, MZ-200, MZ-300, MZ-306X, MZX-303S, MZX-303M, MZX-304OTS, MZ-500HP, MZ-505T, MZX-505HPS, MZY-505M, MZ-506X, MZY-203S, MZX-203OTS, MZY-210M3S, TMZ-HA1, and MZX-5080TS (manufactured by TAYCA Co., Ltd.).

When the microparticulate metal oxide is added to a cosmetic composition including the organopolysiloxane of the present invention, the amount of the microparticulate metal oxide is not particularly limited but is preferably 0.1 to 70 mass%, more preferably 5 to 65 mass%, and still more preferably 45 to 65 mass% relative to the whole amount of the dispersion. If the amount is less than 0.1 mass%, sufficient UV-shielding effects cannot be obtained. If the microparticulate metal oxide is added in an amount larger than 70 mass%, the cosmetic composition may not spread well or may form a white or powdery cosmetic film.

### • Hydrophobized coloring pigments

The coloring pigment for the hydrophobized coloring pigment is not particularly limited and may be any pigment commonly used in cosmetic compositions for the purpose of coloring. Examples include red iron oxide (labeling name, INCI: Iron Oxides), yellow iron oxide (labeling name, INCI: Iron Oxides), white titanium oxide (labeling name, INCI: Titanium Dioxide), black iron oxide (labeling name, INCI: Iron Oxides), ultramarine (labeling name, INCI: Ultramarines), Prussian blue (labeling name, INCI: Ferric Ferrocyanide, Ferric Ammonium Ferrocyanide), manganese violet (labeling name, INCI: Manganese Violet), cobalt titanate (labeling name, INCI: Cobalt Titanium Oxide), chromium hydroxide (labeling name, INCI: Chromium Hydroxide Green), chromium oxide (labeling name, INCI: Chromium Oxide Greens), Al/cobalt oxide (labeling name, INCI: Cobalt Aluminum Oxide), fired titanium/titanium oxide (labeling name, INCI: Titanium/Titanium Dioxide), Li/cobalt titanate (labeling name, INCI: Lithium Cobalt Titanate), sintered iron oxide/titanium oxide (labeling name, Iron Oxide/Titanium Dioxide Sinter), composites doped with a different metal, such as iron oxide-doped titanium oxide (labeling name, INCI: Iron Oxides, Titanium Dioxide), titanium nitride (labeling name, INCI: Titanium Nitride), ferrous hydroxide (labeling name, INCI: Iron Hydroxide), inorganic brown pigments, such as γ-iron oxide, inorganic yellow pigments, such as loess, and colored pigments, such as lake form of tar dyes and lake form of natural dyes. Any of the foregoing may be used.

The hydrophobized coloring pigment may take any shape, with examples including spherical, generally spherical, bar, spindle, petal, strip, and irregular shapes. Its geometry is not particularly limited as long as a color can be imparted to a cosmetic composition. In view of hiding power, a pigment having a particle size, specifically, a volume average particle size in the range of 150 to 600 nm is preferable. The volume average particle size may be measured by TEM or the like. If the average particle size is less than 150 nm, the hiding power is so low that the cosmetic composition may have low coloring efficiency. If the average particle size is larger than 600 nm, the feeling on use may be deteriorated.

The pigment used in the present invention may be treated on part or the entirety of its surface with an inorganic compound, such as alumina (labeling name, INCI: Alumina), Al hydroxide (labeling name, INCI: Aluminum Hydroxide), silica (labeling name, INCI: Silica), or hydrous silica (labeling name, INCI: Hydrated Silica).

The hydrophobizing treatment for the hydrophobized coloring pigment is surface treatment of the coloring pigment with a hydrophobizing agent. The hydrophobizing agent for the surface of the coloring pigment in the present invention is not particularly limited as long as the agent can impart hydrophobicity. Exemplary treating agents include silicone treating agents, waxes, paraffins, organic fluorine compounds, such as perfluoroalkyl phosphate salts, surfactants, amino acids, such as N-acylglutamic acid, and metal soaps, such as Al stearate (labeling name, INCI: Aluminum Stearate) and Mg myristate (labeling name, INCI: Magnesium Myristate).

In particular, silicone treating agents are preferably used. Examples include silanes or silylating agents, such as triethoxycaprylylsilane (labeling name, INCI: Triethoxycaprylylsilane) and trimethoxysilyl dimethicone (labeling name, INCI: Trimethoxysilyl Dimethicone); silicone oils, such as dimethicone (labeling name, INCI: Dimethicone), hydrogen dimethicone (labeling name, INCI: Hydrogen Dimethicone), and triethoxysilylethyl polydimethylsiloxyethyl hexyl dimethicone (labeling name, INCI: Triethoxysilylethyl Polydimethylsiloxyethyl Hexyl Dimethicone); and silicone compounds, such as (acrylates/tridecyl acrylate/triethoxysilylpropyl methacrylate/dimethicone methacrylate) copolymer (labeling name, INCI: Acrylates/Tridecyl Acrylate/Triethoxysilylpropyl Methacrylate/Dimethicone Methacrylate Copolymer), and (acrylates/dimethicone) copolymer (labeling name, INCI: Acrylates/Dimethicone Copolymer). Suitable silicone treating agents are silicone powder treating agents described in JP 3912961. In particular, for example, triethoxysilylethyl polydimethylsiloxyethyl hexyl dimethicone (labeling name, INCI: Triethoxysilylethyl Polydimethylsiloxyethyl Hexyl Dimethicone) that is a dimethylpolysiloxane having a triethoxysilyl group, a polydimethylsiloxyethyl group, and a hexyl group on side chains is effectively used because this compound exerts high affinity even when the dispersion medium in which the highly hydrophobized coloring pigment is dispersed is a mixture of, for example, a silicone and a hydrocarbon. The surface hydrophobizing agents may be used singly, or two or more may be used in combination.

In the present invention, the coloring pigment may be surface-treated with the hydrophobizing agent by any known production method without limitation. The surface treatment methods are largely classified into dry process and wet process. In a dry process, for example, the treatment may be performed by mixing/contacting the coloring pigment to be used in the present invention with the hydrophobizing agent using an appropriate stirring machine, crusher, mixer, disperser or the like, such as a Henschel mixer, a ball mill, a jet mill, a kneader, a planetary mixer, a sand mill, an attritor, a ribbon blender, a dispersing mixer, or a homomixer. In this process, the treatment may be performed while applying energy, such as heat, mechanochemical mechanical force, or overheated steam. The treatment may also be performed in such a manner that the coloring pigment and the hydrophobizing agent are sufficiently mixed/contacted and thereafter energy, such as heat, mechanochemical mechanical force, or overheated steam, is separately applied. In order to enhance the efficiency in dispersing the hydrophobizing agent in the process of mixing/contacting the hydrophobizing agent with the coloring pigment, for example, the hydrophobizing agent may be dissolved or dispersed beforehand in an appropriate amount of water, solvent, or supercritical fluid, and the resulting solution or dispersion may be sprayed to the coloring pigment. In a wet process, the treatment may be performed in such a manner that the coloring pigment and the hydrophobizing agent are dispersed in water, solvent, or supercritical fluid, thereby being mixed/contacted with each other, thereafter the solvent is evaporated, and further energy, such as heat, mechanochemical mechanical force, or overheated steam, is separately applied.

Specific examples of the hydrophobized coloring pigments include KTP-09 series, especially KTP-09W, KTP-09R, KTP-09Y, and KTP-09B (manufactured by Shin-Etsu Chemical Co., Ltd.).

### • Inorganic powders

Examples of the inorganic powders include microparticles of zirconium oxide (labeling name, INCI: Zirconium Dioxide), zinc oxide (labeling name, INCI: Zinc Oxide), cerium oxide (labeling name, INCI: Cerium Oxide), Mg oxide (labeling name, INCI: Magnesium Oxide), Ba sulfate (labeling name, INCI: Barium Sulfate), calcium sulfate (labeling name, INCI: Calcium Sulfate), Mg sulfate (labeling name, INCI: Magnesium Sulfate), Ca carbonate (labeling name, INCI: Calcium Carbonate), Mg carbonate (labeling name, INCI: Magnesium Carbonate), talc (labeling name, INCI: Talc), cleaved talc (labeling name, INCI: Talc), mica (labeling name, INCI: Mica), kaolin (labeling name, INCI: Kaolin), sericite (labeling name, INCI: Mica), synthetic fluorphlogopite (labeling name, INCI: Synthetic Fluorphlogopite), black mica (labeling name, INCI: Biotite), K silicate (labeling name, INCI: Potassium Silicate), silica (labeling name, INCI: Silica), fumed silica, Al silicate (labeling name, INCI: Aluminum Silicate), Mg silicate (labeling name, INCI: Magnesium Silicate), Al/Mg silicate (labeling name, INCI: Magnesium Aluminum Silicate), Ca silicate (labeling name, INCI: Calcium Silicate), Al/Ca/Na silicate (labeling name, INCI: Aluminum Calcium Sodium Silicate), Li/Mg/Na silicate (labeling name, INCI: Lithium Magnesium Sodium Silicate), Na/Mg silicate (labeling name, INCI: Sodium Magnesium Silicate), Ca/Al borosilicate (labeling name, INCI: Calcium Aluminum Borosilicate), Ca/Na borosilicate (labeling name, INCI: Calcium Sodium Borosilicate), hydroxyapatite (labeling name, INCI: Hydroxyapatite), bentonite (labeling name, INCI: Bentonite), montmorillonite (labeling name, INCI: Montmorillonite), hectorite (labeling name, INCI: Hectorite), zeolite (labeling name, INCI: Zeolite), alumina (labeling name, INCI: Alumina), Al hydroxide (labeling name, INCI: Aluminum Hydroxide), boron nitride (labeling name, INCI: Boron Nitride), and glass (labeling name, INCI: Glass). Furthermore, examples of the inorganic coloring pearly pigments include pearly agents, such as titanium oxide-coated mica; and pearly pigments, such as bismuth oxychloride (labeling name, INCI: Bismuth Oxychloride), bismuth oxychloride (labeling name, INCI: Bismuth Oxychloride) coated with titanium oxide (labeling name, INCI: Titanium Dioxide), talc (labeling name, INCI: Talc) coated with titanium oxide (labeling name, INCI: Titanium Dioxide), fish scale flakes, and coloring mica coated with titanium oxide (labeling name, INCI: Titanium Dioxide). These may be untreated or may have undergone well-known surface treatment generally used for cosmetics.

### • Metal powders

Examples of the metal powders include metal microparticles of aluminum (labeling name, INCI: Aluminum Powder), copper (labeling name, INCI: Copper Powder), and silver (labeling name, INCI: Silver Powder).

### • Organic powders

Examples of the organic powders include powders of silicones, polyamides, polyacrylic acid-acrylates, polyesters, polyethylene, polypropylene, polystyrene, styrene-acrylic acid copolymers, divinylbenzene-styrene copolymers, polyurethanes, vinyl resins, urea resins, melamine resins, benzoguanamine, polymethyl benzoguanamine, tetrafluoroethylene, polymethyl methacrylate (such as, for example, polymethyl methacrylate), celluloses, silk, nylons, phenolic resins, epoxy resins, and polycarbonate. In particular, examples of the silicones include silicone resin particles (specifically, polymethylsilsesquioxane (labeling name, INCI: Polymethylsilsesquioxane) and silicone resin-coated silicone rubber powders (specifically, vinyl dimethicone/methicone silsesquioxane crosspolymer (labeling name, INCI: Vinyl Dimethicone/Methicone Silsesquioxane Crosspolymer), (diphenyl dimethicone/vinyl diphenyl dimethicone/silsesquioxane) crosspolymer (labeling name, INCI: Diphenyl Dimethicone/Vinyl Diphenyl Dimethicone/Silsesquioxane Crosspolymer), polysilicone-1 crosspolymer (labeling name, INCI: Polysilicone-1 Crosspolymer), and polysilicone-22 (labeling name, INCI: Polysilicone-22). Examples further include powders of metal soaps, specifically, zinc stearate (labeling name, INCI: Zinc Stearate), Al stearate (labeling name, INCI: Aluminum Stearate), Ca stearate (labeling name, INCI: Calcium Stearate), Mg stearate (labeling name, INCI: Magnesium Stearate), zinc myristate (labeling name, INCI: Zinc Myristate), Mg myristate (labeling name, INCI: Magnesium Myristate), zinc/Na cetylphosphate (labeling name, INCI: Sodium Zinc Cetyl Phosphate), and K cetylphosphate (labeling name, INCI: Potassium Cetyl Phosphate). Examples further include organic colorants, specifically, tar dyes, such as Red #3, Red #104(1) (labeling name, INCI: Red 28, Red 28 Lake), Red #106, Red #201 (labeling name, INCI: Red 6), Red #202 (labeling name, INCI: Red 7), Red #204, Red #205, Red #220 (labeling name, INCI: Red 34), Red #226 (labeling name, INCI: Red 30), Red #227 (labeling name, INCI: Red 33, Red 33 Lake), Red #228 (labeling name, INCI: Red 36), Red #230(1) (labeling name, INCI: Red 22, Red 22 Lake), Red #230(2), Red #401, Red #505, Yellow #4 (labeling name, INCI: Yellow 5), Yellow #5 (labeling name, INCI: Yellow 6, Yellow 6 Lake), Yellow #202(1) (labeling name, INCI: Yellow 8), Yellow #203 (labeling name, INCI: Yellow 10, Yellow 10 Lake), Yellow #204 (labeling name, INCI: Yellow 11), Yellow #401 (labeling name, INCI:), Blue #1 (labeling name, INCI: Blue 1, Blue 1 Lake), Blue #2, Blue #201, Blue #205 (labeling name, INCI: Blue 4), Blue #404, Green #3 (labeling name, INCI: Green 3, Green 3 Lake), Green #201 (labeling name, INCI: Green 5), Green #202 (labeling name, INCI: Green 6), Green #204 (labeling name, INCI: Green 8), Green #205, Orange #201 (labeling name, INCI: Orange 5), Orange #203 (labeling name, INCI: Pigment Orange 5), Orange #204, Orange #205 (labeling name, INCI: Orange 4, Orange 4 Lake), Orange #206 (labeling name, INCI: Orange 10), and Orange #207 (labeling name, INCI: Orange 11); and natural dyes, such as cochineal (labeling name, INCI: Cochineal), laccaic acid (labeling name, INCI: Laccaic Acid), safflower red (labeling name, INCI: Carthamus Tinctorius (Safflower) Flower Extract), red root extract (labeling name, INCI: Lithospermum Officinale Root Extract), gardenia yellow, and gardenia blue (labeling name, INCI: Hydrolyzed Gardenia Florida Extract).

### • Inorganic-organic composite powders

Examples of the inorganic-organic composite powders include composite powders obtained by coating the surface of an inorganic powder with an organic powder by any well-known method.

The amount in which the powder is added is preferably 30 to 90 mass%, more preferably 40 to 85 mass%, and still more preferably 50 to 85 mass% in the dispersion including the organopolysiloxane of the present invention, an oil, and the powder.

### • Oils

The oil is the dispersion medium in which the powder is dispersed by the organopolysiloxane of the present invention. The oil is not particularly limited but is preferably one having a kinematic viscosity of 1 to 100 mm²/s, more preferably 1 to 30 mm²/s, as measured at 25°C with a Cannon-Fenske viscometer in accordance with the method described in JIS Z 8803: 2011. From the point of view of the compatibility with the organopolysiloxane of the present invention, the kinematic viscosity is still more preferably 1 to 20 mm²/s. Specific examples include silicone oils, hydrocarbon oils, ester oils, higher fatty acids, natural oils, and fluorochemical oils.

The use of these oils is not limited to dispersion media for powders, and the oils may be added to cosmetic compositions for various purposes.

### • Silicone oils

Examples of the silicone oils include low- to high-viscosity, linear or branched dimethicones, such as trisiloxane (labeling name, INCI: Trisiloxane), volatile dimethicone (labeling name, INCI: Dimethicone), low-viscosity dimethicone (labeling name, INCI: Dimethicone), cyclotetrasiloxane (labeling name, INCI: Cyclotetrasiloxane), cyclopentasiloxane (labeling name, INCI: Cyclopentasiloxane), cyclohexasiloxane (labeling name, INCI: Cyclohexasiloxane), methyl trimethicone (labeling name, INCI: Methyl Trimethicone), caprylyl methicone (labeling name, INCI: Caprylyl Methicone), phenyl trimethicone (labeling name, INCI: Phenyl Trimethicone), methylphenylpolysiloxane (labeling name, INCI: Diphenyl Dimethicone), diphenylsiloxy phenyl trimethicone (labeling name, INCI: Diphenylsiloxy Phenyl Trimethicone), ethyl methicone (labeling name, INCI: Ethyl Methicone), ethyl trisiloxane (labeling name, INCI: Ethyl Trisiloxane), and hydrogen dimethicone (labeling name, INCI: Hydrogen Dimethicone); amodimethicone (labeling name, INCI: Amodimethicone), and aminopropyl dimethicone (labeling name, INCI: Aminopropyl Dimethicone). Among those described above, some silicone oils are commercially available as, for example, KF-96L-1cs, KF-96L-1.5cs, KF-96L-2cs, KF-96A-6cs, KF-4422, KF-4418, KF-56A, and KF-995 from Shin-Etsu Chemical Co., Ltd. Examples further include silicone rubbers, such as gum-like dimethicone (labeling name, INCI: Dimethicone) having a high degree of polymerization, gum-like amodimethicone (labeling name, INCI: Amodimethicone), and gum-like dimethylsiloxane/methylphenylsiloxane copolymers; cyclic organopolysiloxane solutions of silicone gums and rubbers; amino acid-modified silicones; fluorine-modified silicones; silicone resins; and silicone resin solutions. Examples of commercially available silicone oils include KF-54 and KF-54HV. The organopolysiloxane of the present invention can be used to control the compatibility with these oils so as to prepare cosmetic compositions utilizing phase separation technology.

### • Hydrocarbon oils

Examples of the hydrocarbon oils include linear or branched hydrocarbon oils. The hydrocarbon oils may be volatile or nonvolatile. Specific examples include Olefin Oligomers (INCI), isododecane (labeling name, INCI: Isododecane), dodecane (labeling name, INCI: Dodecane), isohexadecane (labeling name, INCI: Isohexadecane), undecane (labeling name, INCI: Undecane), squalane (labeling name, INCI: Squalane), squalene (labeling name, INCI: Squalene), mineral oil (labeling name, INCI: Mineral Oil), liquid isoparaffin, polyisobutylene (labeling name), hydrogenated polyisobutene (labeling name, INCI: Hydrogenated Polyisobutene), and C13-15 alkane (labeling name, INCI: C13-15 Alkane).

### • Ester oils

The ester oils are liquid oils that are condensation products of a C1-C20 fatty acid and a C1-C20 alcohol and, similarly to the natural oils, may have an animal-derived or plant-derived composition. Examples include monoesters and polyesters, such as diesters and triesters. Specific examples include diisobutyl adipate (labeling name, INCI: Diisobutyl Adipate), dihexyldecyl adipate (labeling name), diheptylundecyl adipate (labeling name, INCI: Diheptylundecyl Adipate), alkylglycol monoisostearates, such as isostearyl isostearate (labeling name, INCI: Isostearyl Isostearate), isocetyl isostearate (labeling name, INCI: Isocetyl Isostearate), trimethylolpropane triisostearate (labeling name, INCI: Trimethylolpropane Triisostearate), glycol diethylhexanoate (labeling name, INCI: Glycol Diethylhexanoate), cetyl ethylhexanoate (labeling name, INCI: Cetyl Ethylhexanoate), triethylhexanoin (labeling name, INCI: Triethylhexanoin), trimethylolpropane triethylhexanoate (labeling name, INCI: Trimethylolpropane Triethylhexanoate), pentaerythrityl tetraethylhexanoate (labeling name, INCI: Pentaerythrityl Tetraethylhexanoate), cetyl octanoate (labeling name, INCI: Cetyl Ethylhexanoate), octyldodecyl esters, such as octyldodecyl stearoyloxystearate (labeling name, INCI: Octyldodecyl Stearoyl Stearate), oleyl oleate (labeling name, INCI: Oleyl Oleate), octyldodecyl oleate (labeling name, INCI: Octyldodecyl Oleate), decyl oleate (labeling name, INCI: Decyl Oleate), neopentyl glycol diethylhexanoate (labeling name, INCI: Neopentyl Glycol Diethylhexanoate), neopentyl glycol dicaprate (labeling name, INCI: Neopentyl Glycol Dicaprate), triethyl citrate (labeling name, INCI: Triethyl Citrate), diethylhexyl succinate (labeling name, INCI: Diethylhexyl Succinate), amyl acetate (labeling name, INCI: Amyl Acetate), ethyl acetate (labeling name, INCI: Ethyl Acetate), butyl acetate (labeling name, INCI: Butyl Acetate), isocetyl stearate (labeling name, INCI: Isocetyl Stearate), butyl stearate (labeling name, INCI: Butyl Stearate), diisopropyl sebacate (labeling name, INCI: Diisopropyl Sebacate), diethylhexyl sebacate (labeling name, INCI: Diethylhexyl Sebacate), cetyl lactate (labeling name, INCI: Cetyl Lactate), myristyl lactate (labeling name, INCI: Myristyl Lactate), isononyl isononanoate (labeling name, INCI: Isononyl Isononanoate), isotridecyl isononanoate (labeling name, INCI: Isotridecyl Isononanoate), coco-alkyl caprylate-caprate (labeling name, INCI: Coco-Caprylate-Caprate), glyceryl tri(caprylate/caprate) (labeling name, INCI: Caprylic/Capric Triglyceride), palmitates, such as isopropyl palmitate (labeling name, INCI: Isopropyl Palmitate), ethylhexyl palmitate (labeling name, INCI: Ethylhexyl Isopalmitate), and hexyldecyl palmitate (labeling name, INCI: Isocetyl Palmitate, Hexyldecyl Palmitate), cholesteryl hydroxystearate (labeling name, INCI: Cholesteryl Hydroxystearate), myristates, such as isopropyl myristate (labeling name, INCI: Isopropyl Myristate), octyldodecyl myristate (labeling name, INCI: Octyldodecyl Myristate), and myristyl myristate (labeling name, INCI: Myristyl Myristate), ethylhexyl laurate (labeling name, INCI: Ethylhexyl Laurate), hexyl laurate (labeling name, INCI: Hexyl Laurate), dioctyldodecyl lauroyl glutamate (labeling name, INCI: Dioctyldodecyl Lauroyl Glutamate), isopropyl lauroyl sarcosinate (labeling name, INCI: Isopropyl Lauroyl Sarcosinate), diisostearyl malate (labeling name, INCI: Diisostearyl Malate), and glyceride oils, such as glyceryl acetate (labeling name, INCI: Glyceryl Acetate) and glyceryl stearate (labeling name, INCI: Glyceryl Stearate).

### • Higher fatty acids

Examples of the higher fatty acids include oleic acid (labeling name, INCI: Oleic Acid), linoleic acid (labeling name, INCI: Linoleic Acid), linolenic acid (labeling name, INCI: Linolenic Acid), arachidonic acid (labeling name, INCI: Arachidonic Acid), eicosapentaenoic acid (EPA) (labeling name, INCI: Eicosapentaenoic Acid), docosahexaenoic acid (DHA) (labeling name, INCI: Docosahexaenoic Acid), isostearic acid (labeling name, INCI: Isostearic Acid), and hydroxystearic acid (labeling name, INCI: Hydroxystearic Acid).

### • Natural oils

Examples of the natural oils include natural animal and plant oils and fats, and semi-synthetic oils and fats. Specific examples include avocado oil (labeling name, INCI: Persea Gratissima (Avocado) Oil), linseed oil (labeling name, INCI: Linum Usitatissimum (Linseed) Seed Oil), almond oil (labeling name, INCI: Prunus Amygdalus Dulcis (Sweet Almond) Oil), olive oil (labeling name, INCI: Olea Europaea (Olive) Fruit Oil), Torreya California oil (labeling name, INCI: Torreya Californica (California Nutmeg) Oil), citronella grass oil (labeling name, INCI: Cymbopogon Nardus (Citronella) Oil), shark liver oil (labeling name, INCI: Shark Liver Oil), cod liver oil (labeling name, INCI: Cod Liver Oil), fish liver oil (labeling name, INCI: Fish Liver Oil), kyounin oil (labeling name, INCI: Kyounin Yu), sesame oil (labeling name, INCI: Sesamum Indicum (Sesame) Seed Oil), rice germ oil (labeling name, INCI: Oryza Sativa (Rice) Germ Oil), rice bran oil (labeling name, INCI: Oryza Sativa (Rice) Bran Oil), camellia oil (labeling name, INCI: Camellia Kissi Seed Oil), turtle oil (labeling name, INCI: Turtle Oil), Japanese camellia oil (labeling name, INCI: Camellia Japonica Seed Oil), primrose oil (labeling name, INCI: Oenothera Biennis (Evening Primrose) Oil), corn germ oil (labeling name, INCI: Zea Mays (Corn) Germ Oil), rapeseed oil (labeling name, INCI: Rape Shushi Yu), wheat germ oil (labeling name, INCI: Triticum Vulgare (Wheat) Germ Oil), persic oil (labeling name, INCI:), palm oil (labeling name, INCI: Elaeis Guineensis (Palm) Oil), palm kernel oil (labeling name, INCI: Elaeis Guineensis (Palm) Kernel Oil), castor oil (labeling name, INCI: Ricinus Communis (Castor) Seed Oil), hydrogenated castor oil (labeling name), sunflower seed oil (labeling name, INCI: Helianthus Annuus (Sunflower) Seed Oil), grape seed oil (labeling name, INCI: Vitis Vinifera (Grape) Seed Oil), jojoba seed oil (labeling name, INCI: Simmondsia Chinensis (Jojoba) Seed Oil), macadamia nut oil (labeling name, INCI: Macadamia Ternifolia Seed Oil), mink oil (labeling name, INCI: Mink Oil), meadowfoam oil (labeling name, INCI: Limnanthes Alba (Meadowfoam) Seed Oil), cotton seed oil (labeling name, INCI: Gossypium Herbaceum (Cotton) Seed Oil), coconut oil (labeling name, INCI: Cocos Nucifera (Coconut) Oil), hydrogenated coconut oil (labeling name, INCI: Hydrogenated Coconut Oil), egg yolk oil (labeling name, INCI: Egg Oil), and Argania Spinosa kernel oil (labeling name, INCI: Argania Spinosa Kernel Oil).

### • Fluorochemical oils

Examples of the fluorochemical oils include perfluoropolyethers, such as polyperfluoromethylisopropyl ether (labeling name, INCI: Polyperfluoromethylisopropyl Ether), and perfluorocarbons, such as perfluorodecalin (labeling name, INCI: Perfluorodecalin) and perfluorohexane (labeling name, INCI: Perfluorohexane).

The amount in which the oil is added is preferably 9 to 69 mass%, more preferably 14 to 50 mass%, and still more preferably 14 to 45 mass% in the dispersion including the organopolysiloxane of the present invention, the oil, and the powder.

### [Methods for producing the dispersion]

The dispersion is preferably prepared in such a manner that the powder is mixed beforehand with the organopolysiloxane of the present invention and the oil. The dispersing machine to be used is selected appropriately from, for example, a three-roll mill, a homomixer, a disperser, a medium agitating mill, such as a bead mill, a wet high-pressure dispersing machine, and an ultrasonic dispersing machine in accordance with the viscosity of the dispersion to be prepared.

### [Cosmetic compositions]

The cosmetic composition is not particularly limited as long as it includes the organopolysiloxane of the present invention. When a powder is added to the cosmetic composition containing the organopolysiloxane of the present invention, the powder may be blended into the cosmetic composition without any pretreatment similarly to other ingredients. When higher dispersibility in the cosmetic composition is desired, it is preferable that the powder be mixed beforehand with other ingredients to form a dispersion, which is then blended into the cosmetic composition.

The cosmetic composition may include one, or a combination of two or more of the organopolysiloxanes of the present invention. As described hereinabove, the organopolysiloxane of the present invention offers excellent powder dispersibility. However, the purpose of use thereof in cosmetic compositions is not limited to the use as a dispersant. For example, the organopolysiloxane may be used as an emulsifier in preparing a water-in-oil emulsion, may be used as a stabilizer in an oil-in-water emulsion, or may be used as a feel improver or a compatibilizer in non-aqueous preparations. The amount in which the organopolysiloxane is added is preferably in the range of 0.1 to 40 mass% of the whole of the cosmetic composition, and, when used as an emulsifier, the amount is more preferably 0.1 to 5 mass% of the whole of the cosmetic composition. When the organopolysiloxane is added in the form of dispersion, the amount of the organopolysiloxane may be controlled appropriately so that the amount in the cosmetic composition will fall within the above range. By virtue of the use of the organopolysiloxane or the dispersion, the cosmetic composition of the present invention excels in feeling on use and spreadability and preferably further in stability.

The cosmetic composition of the present invention may contain, in addition to the oil and the powder described above, various ingredients that are used in conventional cosmetic compositions as long as the advantageous effects of the present invention are not impaired. For example, the cosmetic composition may include (1) oils, (2) aqueous ingredients, (3) surfactants, (4) powders, (5) compositions consisting of a crosslinked organopolysiloxane and an oil that is liquid at room temperature, (6) film-forming agents, and (7) other additives. These may be used singly, or two or more may be used in appropriate combination. The amounts may be appropriately selected. The cosmetic compositions may include the powders and the oils described hereinabove with respect to the dispersions.

### (1) Oils

The oils may be volatile or nonvolatile and may be solid, semi-solid, or liquid at room temperature (25°C). Examples include the oils described hereinabove, for example, the oils used in the dispersions described hereinabove, ingredients that are solid or semi-solid at room temperature (25°C), and UV absorbers.

Examples of the oily ingredients that are solid at 25°C include waxes, hydrocarbons, esters, higher alcohols, and higher fatty acids each having a melting point of preferably 40°C or above, more preferably 60 to 110°C. The oily ingredients are not particularly limited as long as they are commonly blended in cosmetic compositions. Specific examples include plant waxes, such as carnauba wax, sugar cane wax, candelilla wax, purified candelilla wax, rice wax, Japan wax, jojoba wax, kapok wax, rice bran wax, myrica cerifera fruit wax, shea butter, cacao butter, Japan wax (INCI: Rhus Succedanea Fruit Wax), montan wax (INCI: Montan Wax), and hydrogenated castor oil isostearate; animal waxes, such as beeswax, beef tallow, beef bone fat, lard (INCI: Lard), horse fat (INCI: Horse Fat), mutton tallow, lanolin (INCI: Lanolin), Ericerus pela wax, shellac wax, and sperm wax; semi-synthetic waxes, such as lanolin esters, lanolin fatty acid esters, and beeswax acid esters; hydrogenated oils, such as hydrogenated castor oil and hydrogenated coconut oil; hydrocarbon waxes, such as solid paraffin, polyethylene, ceresin, ozokerite, and microcrystalline wax; higher alcohols, such as stearyl alcohol, behenyl alcohol, and cetanol; wax esters, such as synthetic beeswax; amino acid stearyl alcohols, such as dioctyldodecyl lauroylglutamate, dioctyldodecyl lauroylglutamate, and dioctyldodecyl lauroylglutamate; fatty acids, such as stearic acid and behenic acid; silicone waxes, such as acrylate silicone resins in the form of acrylate silicone graft or block copolymers (such as acrylate silicone graft copolymer: KP-561P manufactured by Shin-Etsu Chemical Co., Ltd.); and derivatives thereof. Any one, or two or more selected from the foregoing are preferable.

Examples of the UV absorbers include oxybenzone-1 (labeling name, INCI: Benzophenone-1), oxybenzone-2 (labeling name, INCI: Benzophenone-2), oxybenzone-3 (labeling name, INCI: Benzophenone-3), oxybenzone-4 (labeling name, INCI: Benzophenone-4), oxybenzone-5 (labeling name, INCI: Benzophenone-5), oxybenzone-6 (labeling name, INCI: Benzophenone-6), oxybenzone-9 (labeling name, INCI: Benzophenone-9), Homosalate (INCI), Octocrylene (INCI), t-butyl methoxydibenzoylmethane (labeling name, INCI: Butyl Methoxydibenzoylmethane), ethylhexyl salicylate (labeling name, INCI: Ethylhexyl Salicylate), diethylaminohydroxybenzoyl hexyl benzoate (labeling name, INCI: Diethylamino Hydroxybenzoyl Hexyl Benzoate), Polysilicone-15 (INCI), dimethoxybenzylidene dioxoimidazolidine octyl propionate (labeling name, INCI: Ethylhexyl Dimethoxybenzylidene Dioxoimidazolidine Propionate), terephthalylidene dicamphor sulfonic acid (labeling name, INCI: Terephthalylidene Dicamphor Sulfonic Acid), Ethylhexyl Triazone (INCI), methylbis(trimethylsiloxy)silyl isopentyl trimethoxycinnamate (labeling name, INCI: Isopentyl Trimethoxycinnamate Trisiloxane), Drometrizole Trisiloxane (INCI), dimethyl PABA ethylhexyl (labeling name, INCI: Ethylhexyl Dimethyl PABA), isopropyl para-methoxycinnamate (labeling name, INCI: Isopropyl Methoxycinnamate), ethylhexyl methoxycinnamate (labeling name, INCI: Ethylhexyl Methoxycinnamate), Bisethylhexyloxyphenol Methoxyphenyltriazine (INCI), phenylbenzimidazole sulfonic acid (labeling name, INCI: Phenylbenzimidazole Sulfonic Acid), Methylene Bis-Benzotriazolyl Tetramethylbutylphenol (INCI), glyceryl ethylhexanoate dimethoxycinnamate (labeling name, INCI: Glyceryl Ethylhexanoate Dimethoxycinnamate), Glyceryl PABA (INCI), methyl diisopropylcinnamate (labeling name, INCI: Diisopropyl Methyl Cinnamate), Cinoxate (INCI), and ethylhexyl dimethoxybenzylidene dioxoimidazolidine propionate (labeling name, INCI: Ethylhexyl Dimethoxybenzylidene Dioxoimidazolidine Propionate). Furthermore, a UVA absorber (such as, for example, Diethylamino Hydroxybenzoyl Hexyl Benzoate) and a UVB absorber (such as, for example, Ethylhexyl Methoxycinnamate) may be used in combination, or absorbers belonging to the same type may be combined appropriately.

The amount in which the oil is added is preferably 1 to 85 mass%, more preferably 3 to 30 mass%, and still more preferably 5 to 20 mass% in the cosmetic composition.

### (2) Aqueous ingredients

The aqueous ingredients are not particularly limited as long as they are aqueous ingredients that are usually added to cosmetic compositions. Specific examples include water, lower alcohols preferably having 2 to 5 carbon atoms, such as ethanol (labeling name, INCI: Alcohol) and isopropanol (labeling name, INCI: Isopropyl Alcohol), and sugar alcohols, such as Sorbitol (INCI), Maltose (INCI), and Xylitol (INCI). Examples further include polyhydric alcohols, such as BG (labeling name, INCI: Butylene Glycol), PG (labeling name, INCI: Propylene Glycol), DPG (labeling name, INCI: Dipropylene Glycol), Pentylene Glycol (INCI), 1,10-Decanediol (INCI), Octanediol (INCI), 1,2-Hexanediol (INCI), Erythritol (INCI), Glycerin (INCI), Diglycerin (INCI), and polyethylene glycol; and moisturizers, such as Glucose (INCI), Glyceryl Glucoside (INCI), Betaine (INCI), Na chondroitin sulfate (labeling name, INCI: Sodium Chondroitin Sulfate), PCA-Na (labeling name, INCI: Sodium PCA), Methyl Gluceth-10 (INCI), Methyl Gluceth-20 (INCI), hyaluronic acid, egg yolk lecithin, soybean lecithin, phosphatidylcholine, phosphatidylethanolamine, phosphatidylserine, phosphatidylglycerol, phosphatidylinositol, and sphingophospholipid. The amount of water, etc. may be the balance.

### (3) Surfactants

The surfactants may be nonionic, anionic, cationic, or ampholytic. The surfactants are not particularly limited and any surfactants usually used in cosmetic compositions may be used. Among such surfactants, one, or two or more selected from non-crosslinked silicone surfactants and crosslinked silicone surfactants are preferable because stable cosmetic compositions are obtained. For any types of surfactants, the amount is preferably 0.1 to 20 mass% of the whole of the cosmetic composition. An amount of 0.1 mass% or more ensures that the dispersing and emulsifying functions are fully achieved whereas an amount of 20 mass% or less eliminates the risk that the cosmetic composition gives a sticky feeling on use. The HLB of the surfactants is not limited but is preferably 2 to 14.5 in order to maintain the water resistance of the cosmetic composition.

The non-crosslinked silicone surfactants are linear or branched silicones in which some methyl groups on the backbone are substituted with hydrophilic groups, such as polyethylene glycol and polyglycerin. Specifically, preferred non-crosslinked silicone surfactants are linear or branched polyoxyethylene-modified organopolysiloxanes, linear or branched polyoxyethylene/polyoxypropylene-modified organopolysiloxanes, linear or branched polyoxyethylene/alkyl-co-modified organopolysiloxanes, linear or branched polyoxyethylene/polyoxypropylene/alkyl-co-modified organopolysiloxanes, linear or branched polyglycerin-modified organopolysiloxanes, linear or branched polyglycerin/alkyl-co-modified organopolysiloxanes, and linear or branched pyrrolidone-modified organopolysiloxanes. Examples include PEG-11 Methyl Ether Dimethicone (INCI), PEG/PPG-20/22 Butyl Ether Dimethicone (INCI), PEG-3 Dimethicone (INCI), PEG-10 Dimethicone (INCI), PEG-9 Polydimethylsiloxyethyl Dimethicone (INCI), Lauryl PEG-9 Polydimethylsiloxyethyl Dimethicone (INCI), Cetyl PEG/PPG-10/1 Dimethicone (INCI), Polyglyceryl-3 Disiloxanedimethicone (INCI), Polyglyceryl-3 Polydimethylsiloxyethyl Dimethicone (INCI), Lauryl Polyglyceryl-3 Polydimethylsiloxyethyl Dimethicone (INCI), and Bis-Butyldimethicone Polyglyceryl-3 (INCI).

Examples of commercially available products include KF-6011, KF-6011P, KF-6012, KF-6015, KF-6017, KF-6043, KF-6028, KF-6038, KF-6048, KF-6100, KF-6104, KF-6106, KF-6105, and KF-6115 manufactured by Shin-Etsu Chemical Co., Ltd.

Examples of the crosslinked silicone surfactants include crosslinked polyether-modified silicones, such as (Dimethicone/(PEG-10/15)) Crosspolymer (INCI), (PEG-15/Lauryl Dimethicone) Crosspolymer (INCI), (PEG-10/Lauryl Dimethicone) Crosspolymer (INCI), and (PEG-15/Lauryl Polydimethylsiloxyethyl Dimethicone) Crosspolymer (INCI); and crosslinked polyglycerin-modified silicones, such as (Dimethicone/Polyglycerin-3) Crosspolymer (INCI), (Lauryl Dimethicone/Polyglycerin-3) Crosspolymer (INCI), and (Polyglycerin-3/Lauryl Polydimethylsiloxyethyl Dimethicone) Crosspolymer (INCI).

When the crosslinked silicone surfactant is used as a composition consisting of the crosslinked silicone surfactant and an oil that is liquid at room temperature, it is preferable that the crosslinked silicone surfactant be swollen by containing its own weight or more of the liquid oil.

The liquid oil may be any of the liquid silicone oils, the hydrocarbon oils, the ester oils, the natural animal and plant oils, the semi-synthetic oils, and the fluorochemical oils described hereinabove with respect to the oils. Examples include Cyclopentasiloxane (INCI), Dimethicone (INCI), Mineral Oil (INCI), Isododecane (INCI), Isohexadecane (INCI), Triethylhexanoin (INCI), isotridecyl isononanoate (labeling name, INCI: Isotridecyl Isononanoate), and Squalane (INCI). Commercially available crosslinked silicone surfactants swollen by containing a liquid oil include, for example, KSG-210, KSG-240, KSG-270, KSG-310, KSG-320, KSG-330, KSG-340, KSG-320Z, KSG-350Z, KSG-710, KSG-810, KSG-820, KSG-830, KSG-840, KSG-820Z, and KSG-850Z manufactured by Shin-Etsu Chemical Co., Ltd.

### (4) Powders

The powder may be added as a dispersion together with the organopolysiloxane or may be added separately. Furthermore, the powder may be added to the cosmetic composition as other dispersion. Specific examples of dispersions in which UV-absorbing and scattering particles are dispersed in an oil include SPD series (product name) manufactured by Shin-Etsu Chemical Co., Ltd., in particular, SPD-T5, T5L, Z5, Z5L, T6, Z6, T7, and Z7L.

The amount in which the powder is added is preferably 0.5 to 90 mass%, more preferably 1 to 30 mass%, and still more preferably 5 to 20 mass% in the cosmetic composition.

### (5) Compositions consisting of a crosslinked organopolysiloxane and an oil that is liquid at room temperature

In the composition consisting of a crosslinked organopolysiloxane and an oil that is liquid at room temperature, it is preferable that the crosslinked organopolysiloxane be swollen by containing its own weight or more of the liquid oil. The liquid oil may be any of the liquid silicone oils, the hydrocarbon oils, the ester oils, the natural animal and plant oils, the semi-synthetic oils, and the fluorochemical oils described hereinabove with respect to the oils. Examples include Cyclopentasiloxane (INCI), Dimethicone (INCI), Mineral Oil (INCI), Isododecane (INCI), Isohexadecane (INCI), Triethylhexanoin (INCI), isotridecyl isononanoate (labeling name, INCI: Isotridecyl Isononanoate), and Squalane (INCI).

Unlike the crosslinked silicone surfactants described above as the ingredients (3), the ingredients (5) are compounds that do not have a polyether or polyglycerin structure in the molecular structure. Specific examples include Dimethicone/Vinyl Dimethicone Crosspolymer (INCI), Dimethicone/Phenyl Vinyl Dimethicone Crosspolymer (INCI), Vinyl Dimethicone/Lauryl Dimethicone Crosspolymer (INCI), and Lauryl Polydimethylsiloxyethyl Dimethicone/Bis-Vinyl Dimethicone Crosspolymer (INCI).

Examples of commercially available compositions consisting of a crosslinked organopolysiloxane and an oil that is liquid at room temperature include KSG-15, KSG-1510, KSG-16, KSG-1610, KSG-19, KSG-016F, KSG-18A, KSG-41A, KSG-42A, KSG-43, KSG-44, KSG-042Z, KSG-045Z, and KSG-048Z manufactured by Shin-Etsu Chemical Co., Ltd.

### (6) Film-forming agents

The film-forming agent is added mainly for the purpose of further extending the durability of the effects of the cosmetic composition. The film-forming agents are not particularly limited. Silicone compositions are preferable from the point of view of imparting water repellency. Specifically, for example, use may be made of trimethylsiloxysilicic acid, crosslinked trimethylsiloxysilicic acid, modified trimethylsiloxysilicic acid, acrylic-silicone film formers, silicone-modified norbornene, silicone-modified pullulan, and silicone-modified polyvinyl alcohol. Examples of the film-forming agents in the form of silicone compositions include trimethylsiloxysilicic acid (labeling name, INCI: Trimethylsiloxysilicate), Acrylate/Dimethicone Copolymer (INCI), Norbornene/Tris(Trimethylsiloxy)Silylnorbornene Copolymer (INCI), and pullulan tri(trimethylsiloxy)silylpropyl carbamide (labeling name, INCI: Trimethylsiloxysilylcarbamoyl Pullulan).

The film-forming agent may be previously dissolved into an oil that is liquid at room temperature before being added to the cosmetic composition. The liquid oil may be any of the liquid silicone oils, the hydrocarbon oils, the ester oils, the natural animal and plant oils, the semi-synthetic oils, and the fluorochemical oils described hereinabove with respect to the oils (1) that are optional ingredients.

Specific examples of commercially available silicone film-forming agents include KF-7312J, KP-545, KP-549, KP-543, NBN-30-ID, TSPL-30-ID, and TSPL-30-D5 manufactured by Shin-Etsu Chemical Co., Ltd.

### (7) Other additives

Other additives include oil-soluble gelling agents, preservatives, antibacterial agents, antiperspirants, fragrances, salts, antioxidants, acidity regulators, chelating agents, refreshing agents, anti-inflammatory agents, skin modifying ingredients (such as brightening or whitening agents, cell activators, anti-skin-roughening agents, blood flow enhancing agents, skin astringents, and antiseborrheic agents), vitamins, amino acids, nucleic acids, hormones, and clathrate compounds.

### • Oil-soluble gelling agents

Exemplary oil-soluble gelling agents include metal soaps, such as aluminum stearate, magnesium stearate, and zinc myristate; amino acid derivatives, such as lauroyl glutamic acid (labeling name, INCI: Lauroyl Glutamic Acid) and α,γ-di-n-butylamine; dextrin fatty acid esters, such as dextrin palmitate (labeling name, INCI: Dextrin Palmitate), dextrin isostearate (labeling name, INCI: Dextrin Isostearate), dextrin myristate (labeling name, INCI: Dextrin Myristate), inulin stearate (labeling name, INCI: Stearoyl Inulin), and dextrin palmitate/ethylhexanoate (labeling name, INCI: Dextrin Palmitate/Ethylhexanoate); sucrose fatty acid esters, such as sucrose palmitate and sucrose stearate; fructooligosaccharide fatty acid esters, such as fructooligosaccharide stearate and fructooligosaccharide 2-ethylhexanoate; benzylidene derivatives of sorbitol, such as monobenzylidene sorbitol and dibenzylidene sorbitol; organo-modified clay minerals of hectorite, such as Disteardimonium Hectorite (INCI) and Stearalkonium Hectorite (INCI); and Stearalkonium Bentonite (INCI).

### • Preservatives and antibacterial agents

Examples of the preservatives and the antibacterial agents include alkyl p-hydroxybenzoates, benzoic acid, sodium benzoate, sorbic acid, potassium sorbate, phenoxyethanol, imidazolidinyl urea, salicylic acid, isopropyl methyl phenol, phenol, p-chloro-m-cresol, hexachlorophene, benzalkonium chloride, chlorohexidine chloride, trichlorocarbanilide, iodopropynyl butylcarbamate, polylysine, photosensitizers, silver, and plant extracts.

### • Antiperspirants

Examples of the antiperspirants include aluminum halohydrates, such as aluminum chlorohydrate, aluminum halides, such as aluminum chloride, aluminum allantoinate, tannic acid, persimmon tannin, aluminum potassium sulfate, zinc oxide, zinc p-phenolsulfonate, calcined alum, aluminum zirconium tetrachlorohydrate, and aluminum zirconium trichlorohydrex glycine. In particular, preferred ingredients that exert a higher effect include aluminum halohydrates, aluminum halides, and complexes or mixtures thereof with zirconium oxyhalides and zirconium hydroxyhalides (for example, aluminum zirconium tetrachlorohydrate and aluminum zirconium trichlorohydrex glycine).

### • Fragrances

The fragrances include natural fragrances and synthetic fragrances. Examples of the natural fragrances include plant fragrances separated from flowers, leaves, stems, and pericarps; and animal fragrances, such as musk and civet. Examples of the synthetic fragrances include hydrocarbons, such as monoterpene; alcohols, such as aliphatic alcohols and aromatic alcohols; aldehydes, such as terpene aldehydes and aromatic aldehydes; ketones, such as alicyclic ketones; esters, such as terpene esters; lactones; phenols; oxides; nitrogen-containing compounds; and acetals.

### • Salts

Examples of the salts include inorganic salts, organic acid salts, amine salts, and amino acid salts. Exemplary inorganic salts include sodium salts, potassium salts, magnesium salts, calcium salts, aluminum salts, zirconium salts, and zinc salts of inorganic acids, such as hydrochloric acid, sulfuric acid, carbonic acid, and nitric acid. Exemplary organic acid salts include salts of organic acids, such as acetic acid, dehydroacetic acid, citric acid, malic acid, succinic acid, ascorbic acid, and stearic acid. Exemplary amine salts and amino acid salts include salts of amines, such as triethanol amine, and salts of amino acids, such as glutamic acid. Also useful are salts of hyaluronic acid, chondroitin sulfate, aluminum zirconium glycine complex, and acid-alkali neutral salts used in cosmetic formulations.

### • Antioxidants

Examples of the antioxidants include, but are not particularly limited to, carotenoid, ascorbic acid and salts thereof, ascorbyl stearate, tocopherol, tocopherol acetate, tocopherol, p-t-butylphenol, butyl hydroxyanisole, dibutyl hydroxytoluene, phytic acid, ferulic acid, thiotaurine, hypotaurine, sulfites, erythorbic acid and salts thereof, chlorogenic acid, epicatechin, epigallocatechin, epigallocatechin gallate, apigenin, kaempferol, myricetin, and quercetin. The antioxidants may be used singly, or two or more may be used in combination.

### • Acidity regulators

Examples of the acidity regulators include lactic acid, citric acid, glycolic acid, succinic acid, tartaric acid, dl-malic acid, potassium carbonate, sodium hydrogencarbonate, and ammonium hydrogencarbonate.

### • Chelating agents

Examples of the chelating agents include alanine, sodium EDTA, sodium polyphosphate, sodium metaphosphate, and phosphoric acid.

### • Refreshing agents

Examples of the refreshing agents include L-menthol, camphor, and menthyl lactate.

### • Anti-inflammatory agents

Examples of the anti-inflammatory agents include allantoin, glycyrrhizinic acid and salts thereof, glycyrrhetinic acid, stearyl glycyrrhetinate, tranexamic acid, and azulene.

### • Skin modifying ingredients

Examples of the skin modifying ingredients include brightening or whitening agents, such as placenta extract, arbutin, glutathione, and Saxifraga Sarmentosa Extract; cell activators, such as royal jelly, photosensitizers, cholesterol derivatives, and bovine blood extracts; anti-skin-roughening agents; blood flow enhancing agents, such as vanillylamide nonylate, benzyl nicotinate, β-butoxyethyl nicotinate, capsaicin, zingerone, cantharides tincture, ichthammol, caffeine, tannic acid, α-borneol, tocopherol nicotinate, inositol hexanicotinate, cyclandelate, cinnarizine, tolazoline, acetylcholine, verapamil, cepharanthine, and γ-oryzanol; skin astringents, such as zinc oxide and tannic acid; and antiseborrheic agents, such as sulfur and thianthol.

### • Vitamins

Examples of the vitamins include vitamin A family, such as vitamin A oil, retinol, retinol acetate, and retinol palmitate; vitamin B family, specifically, vitamin B2 family, such as riboflavin, riboflavin butyrate, and flavin adenine nucleotide, vitamin B6 family, such as pyridoxine hydrochloride, pyridoxine dioctanoate, and pyridoxine tripalmitate, vitamin B12 and derivatives thereof, and vitamin B15 and derivatives thereof; vitamin C family, such as L-ascorbic acid, L-ascorbic acid dipalmitic acid ester, L-ascorbic acid-2-sodium sulfate, and dipotassium L-ascorbic acid phosphoric acid diester; vitamin D family, such as ergocalciferol and cholecalciferol; vitamin E family, such as α-tocopherol, β-tocopherol, γ-tocopherol, dl-α-tocopherol acetate, dl-α-tocopherol nicotinate, and dl-α-tocopherol succinate; nicotinic acids, such as nicotinic acid, benzyl nicotinate, and nicotinic amide; vitamin H; vitamin P; pantothenic acids, such as calcium pantothenate, D-pantothenyl alcohol, pantothenyl ethyl ether, and acetylpantothenyl ethyl ether; and biotin.

### • Amino acids

Examples of the amino acids include glycine, valine, leucine, isoleucine, serine, threonine, phenylalanine, arginine, lysine, aspartic acid, glutamic acid, cystine, cysteine, methionine, and tryptophan.

### • Nucleic acids

Examples of the nucleic acids include deoxyribonucleic acid.

### • Hormones

Examples of the hormones include estradiol and ethenyl estradiol.

### • Clathrate compounds

Examples of the clathrate compounds include cyclodextrin.

The present invention is applicable to a variety of cosmetic compositions, particularly preferably to cosmetic compositions externally applied to the skin, such as skin care cosmetic compositions, makeup cosmetic compositions, antiperspirant cosmetic compositions, and UV-protecting cosmetic compositions, to cosmetic compositions externally applied to the hair, such as hair care cosmetic compositions, and to nail cosmetic compositions. Exemplary skin care cosmetic compositions include skin lotions, milky lotions, creams, cleansing agents, packs, oil liquids, massage creams, cosmetic liquids, cosmetic oils, detergents, deodorants, hand creams, lip creams, and anti-wrinkle agents. Exemplary makeup cosmetic compositions include makeup bases, foundations, concealers, cosmetic powders, cheek colors, eye colors, eye shadows, mascaras, eye liners, eyebrows, and lipsticks. Exemplary antiperspirant cosmetic compositions include antiperspirants of roll-on, cream, solution, and stick types. Exemplary UV-protecting cosmetic compositions include sunscreen oils, sunscreen milky lotions, and sunscreen creams. Exemplary hair care cosmetic compositions include shampoos, conditioners, treatments, and setting agents.

The type of the cosmetic composition of the present invention may be any of, for example, powder, oily liquid, water-in-oil emulsion, oil-in-water emulsion, non-aqueous emulsion, and multi-phase emulsion, such as W/O/W or O/W/O. The form of the cosmetic composition of the present invention may be selected from various forms including liquid, milky lotion, cream, solid, paste, gel, powder, pressed, multilayer, mousse, spray, stick, and pencil.

### EXAMPLES

Examples and Comparative Examples of the present invention are shown below by way of illustration and not by way of limitation. In Examples, the compositional "%" is mass% unless otherwise stated, and the amounts are the amounts of the products described.

### [Example 1]

A reactor was charged with 50 g of triglycerin diallyl ether represented by formula (E-1) below, 128.7 g of an organohydrogenpolysiloxane represented by formula (E-2) below (hydrosilyl groups/allyl groups = 2.0/2.0 [by mol]), 89.3 g of isopropyl alcohol, and 0.04 g of a 3% ethanol solution of platinum-1,3-divinyl-1,1,3,3-tetramethyldisiloxane complex. The reaction was carried out by performing heating at 80°C for 5 hours. Subsequently, 15.4 g of 0.01 N (mol/L) hydrochloric acid was added and the mixture was heated at 80°C for 3 hours to perform hydrolysis. The system was then neutralized with 0.2 g of a 5% aqueous sodium hydrogencarbonate solution. The reaction solution was heated under reduced pressure to distill off the solvent, and the residue was filtered to give the target product (organopolysiloxane) as a fluid and viscous liquid represented by formula (E-3) below.

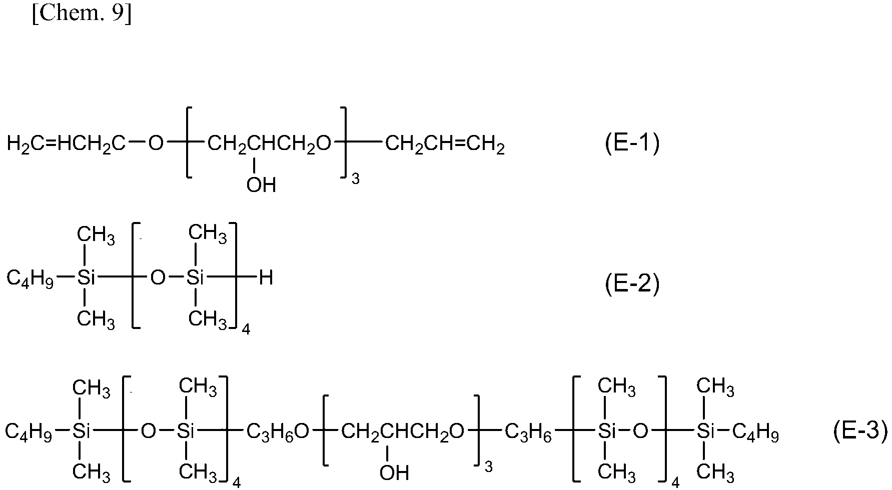

### [Example 2]

A reactor was charged with 50 g of triglycerin diallyl ether represented by formula (E-1) above, 244.8 g of an organohydrogenpolysiloxane represented by formula (E-4) below (hydrosilyl groups/allyl groups = 2.0/2.0 [by mol]), 88.4 g of isopropyl alcohol, and 0.03 g of a 3% ethanol solution of platinum-1,3-divinyl-1,1,3,3-tetramethyldisiloxane complex. The reaction was carried out by performing heating at 80°C for 5 hours. Subsequently, 29.4 g of 0.01 N hydrochloric acid was added and the mixture was heated at 80°C for 3 hours to perform hydrolysis. The system was then neutralized with 0.4 g of a 5% aqueous sodium hydrogencarbonate solution. The reaction solution was heated under reduced pressure to distill off the solvent, and the residue was filtered to give the target product (organopolysiloxane) as a fluid and viscous liquid represented by formula (E-5) below.

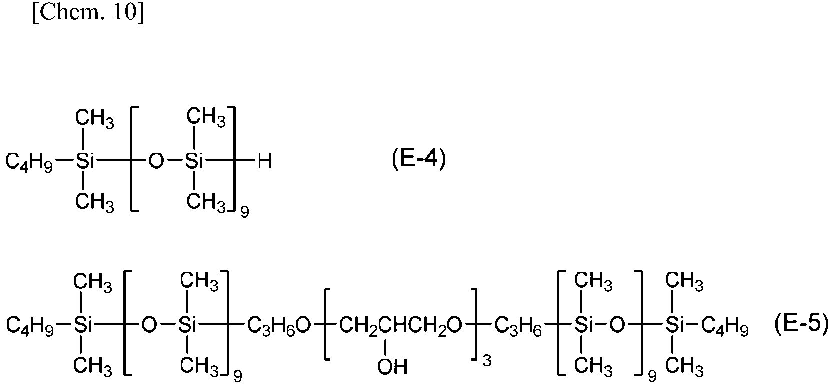

### [Example 3]

A reactor was charged with 50 g of triglycerin diallyl ether represented by formula (E-1) above, 729.2 g of an organohydrogenpolysiloxane represented by formula (E-6) below (hydrosilyl groups/allyl groups = 1.0/2.0 [by mol]), 389.6 g of isopropyl alcohol, and 0.16 g of a 3% ethanol solution of platinum-1,3-divinyl-1,1,3,3-tetramethyldisiloxane complex. The reaction was carried out by performing heating at 80°C for 5 hours. Subsequently, the reactor was charged with 122.4 g of an organohydrogenpolysiloxane represented by formula (E-4) above (hydrosilyl groups/allyl groups = 1.0/2.0 [by mol]), 61.2 g of isopropyl alcohol, and 0.02 g of a 3% ethanol solution of platinum-1,3-divinyl-1,1,3,3-tetramethyldisiloxane complex. The reaction was carried out by performing heating at 80°C for 3 hours. Subsequently, 102.2 g of 0.01 N hydrochloric acid was added and the mixture was heated at 80°C for 3 hours to perform hydrolysis. The system was then neutralized with 1.5 g of a 5% aqueous sodium hydrogencarbonate solution. The reaction solution was heated under reduced pressure to distill off the solvent, and the residue was filtered to give the target product (organopolysiloxane) as a fluid and viscous liquid represented by formula (E-7) below.

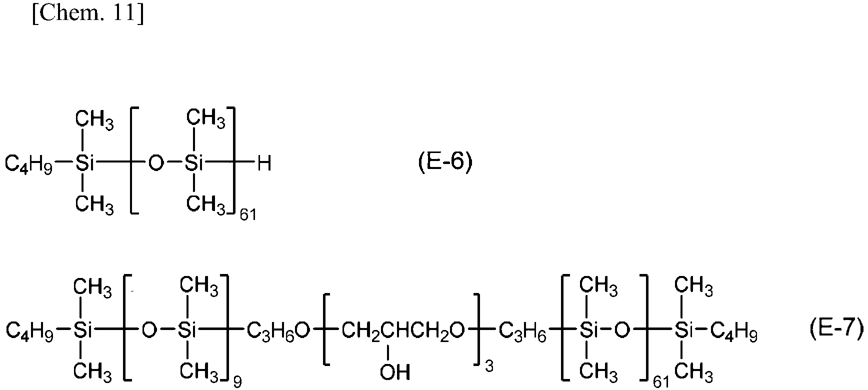

### [Example 4]

A reactor was charged with 50 g of diglycerin diallyl ether represented by formula (E-8) below, 248.8 g of an organohydrogenpolysiloxane represented by formula (E-9) below (hydrosilyl groups/allyl groups = 1.0/2.0 [by mol]), 119.5 g of isopropyl alcohol, and 0.07 g of a 3% ethanol solution of platinum-1,3-divinyl-1,1,3,3-tetramethyldisiloxane complex. The reaction was carried out by performing heating at 80°C for 5 hours. Subsequently, the reactor was charged with 30.1 g of an organohydrogenpolysiloxane represented by formula (E-10) below (hydrosilyl groups/allyl groups = 1.0/2.0 [by mol]), 12.0 g of isopropyl alcohol, and 0.01 g of a 3% ethanol solution of platinum-1,3-divinyl-1,1,3,3-tetramethyldisiloxane complex. The reaction was carried out by performing heating at 80°C for 3 hours. Subsequently, 33.5 g of 0.01 N hydrochloric acid was added and the mixture was heated at 80°C for 3 hours to perform hydrolysis. The system was then neutralized with 0.5 g of a 5% aqueous sodium hydrogencarbonate solution. The reaction solution was heated under reduced pressure to distill off the solvent, and the residue was filtered to give the target product (organopolysiloxane) as a fluid and viscous liquid represented by formula (E-11) below.

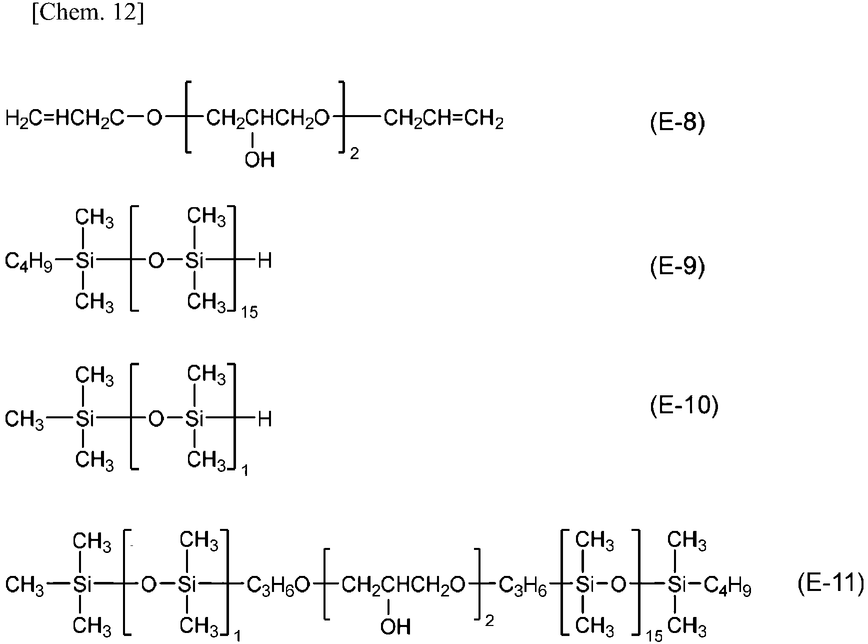

### [Example 5]

A reactor was charged with 50 g of pentaglycerin triallyl ether represented by formula (E-12) below, 330.4 g of an organohydrogenpolysiloxane represented by formula (E-13) below (hydrosilyl groups/allyl groups = 2.0/3.0 [by mol]), 304.3 g of isopropyl alcohol, and 0.13 g of a 3% ethanol solution of platinum-1,3-divinyl-1,1,3,3-tetramethyldisiloxane complex. The reaction was carried out by performing heating at 80°C for 5 hours. Subsequently, the reactor was charged with 36.6 g of an organohydrogenpolysiloxane represented by formula (E-14) (hydrosilyl groups/allyl groups = 1.0/3.0 [by mol]), 29.3 g of isopropyl alcohol, and 0.01 g of a 3% ethanol solution of platinum-1,3-divinyl-1,1,3,3-tetramethyldisiloxane complex. The reaction was carried out by performing heating at 80°C for 3 hours. Subsequently, 44.0 g of 0.01 N hydrochloric acid was added and the mixture was heated at 80°C for 3 hours to perform hydrolysis. The system was then neutralized with 0.7 g of a 5% aqueous sodium hydrogencarbonate solution. The reaction solution was heated under reduced pressure to distill off the solvent, and the residue was filtered to give the target product (organopolysiloxane) as a fluid and viscous liquid represented by formula (E-15) below.

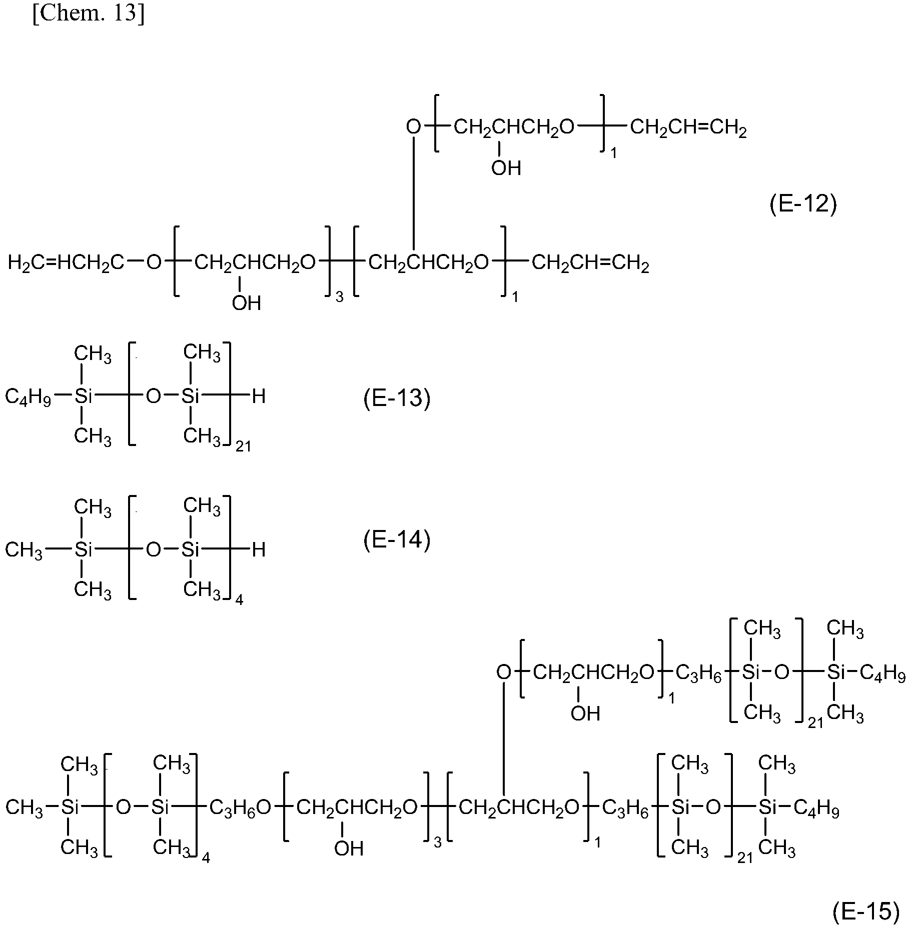

### [Example 6]

A reactor was charged with 50 g of tetraglycerin dioctenyl ether represented by formula (E-16) below, 566.9 g of an organohydrogenpolysiloxane represented by formula (E-17) below (hydrosilyl groups/octenyl groups = 1.0/2.0 [by mol]), 370.1 g of isopropyl alcohol, and 0.10 g of a 3% ethanol solution of platinum-1,3-divinyl-1,1,3,3-tetramethyldisiloxane complex. The reaction was carried out by performing heating at 80°C for 5 hours. Subsequently, the reactor was charged with 61.7 g of an organohydrogenpolysiloxane represented by formula (E-18) below (hydrosilyl groups/allyl groups = 1.0/2.0 [by mol]), 37.0 g of isopropyl alcohol, and 0.01 g of a 3% ethanol solution of platinum-1,3-divinyl-1,1,3,3-tetramethyldisiloxane complex. The reaction was carried out by performing heating at 80°C for 3 hours. Subsequently, 75.4 g of 0.01 N hydrochloric acid was added and the mixture was heated at 80°C for 3 hours to perform hydrolysis. The system was then neutralized with 1.1 g of a 5% aqueous sodium hydrogencarbonate solution. The reaction solution was heated under reduced pressure to distill off the solvent, and the residue was filtered to give the target product (organopolysiloxane) as a fluid and viscous liquid represented by formula (E-19) below.

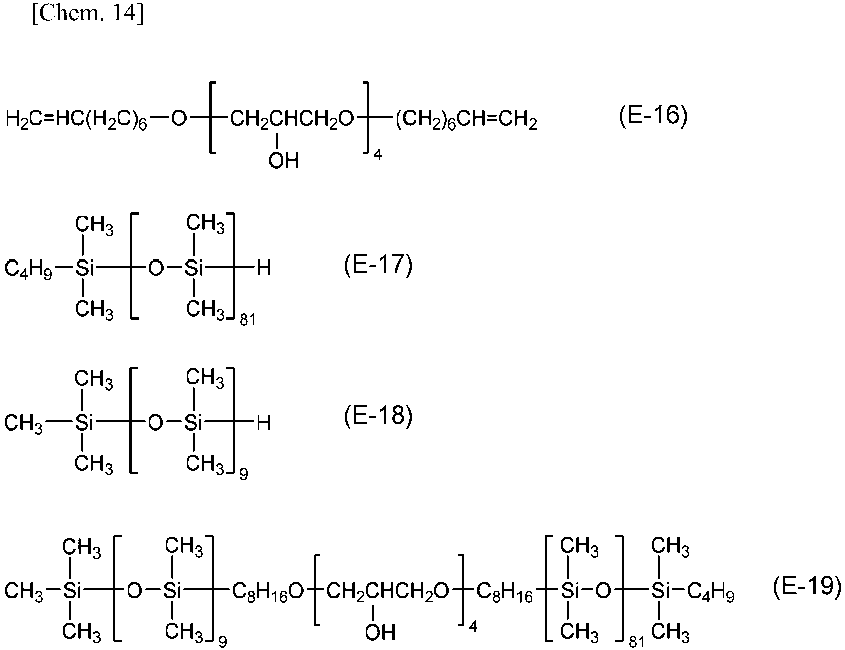

### [Example 7]

A reactor was charged with 50 g of tetraglycerin triallyl ether represented by formula (E-20) below, 730.3 g of an organohydrogenpolysiloxane represented by formula (E-21) below (hydrosilyl groups/allyl groups = 2.0/3.0 [by mol]), 234.1 g of isopropyl alcohol, and 0.13 g of a 3% ethanol solution of platinum-1,3-divinyl-1,1,3,3-tetramethyldisiloxane complex. The reaction was carried out by performing heating at 80°C for 5 hours. Subsequently, the reactor was charged with 82.0 g of an organohydrogenpolysiloxane represented by formula (E-22) below (hydrosilyl groups/allyl groups = 1.0/3.0 [by mol]), 24.6 g of isopropyl alcohol, 0.01 g of a 3% ethanol solution of platinum-1,3-divinyl-1,1,3,3-tetramethyldisiloxane complex. The reaction was carried out by performing heating at 80°C for 3 hours. Subsequently, 97.5 g of 0.01 N hydrochloric acid was added and the mixture was heated at 80°C for 3 hours to perform hydrolysis. The system was then neutralized with 1.5 g of a 5% aqueous sodium hydrogencarbonate solution. The reaction solution was heated under reduced pressure to distill off the solvent, and the residue was filtered to give the target product (organopolysiloxane) as a fluid and viscous liquid represented by formula (E-23) below.

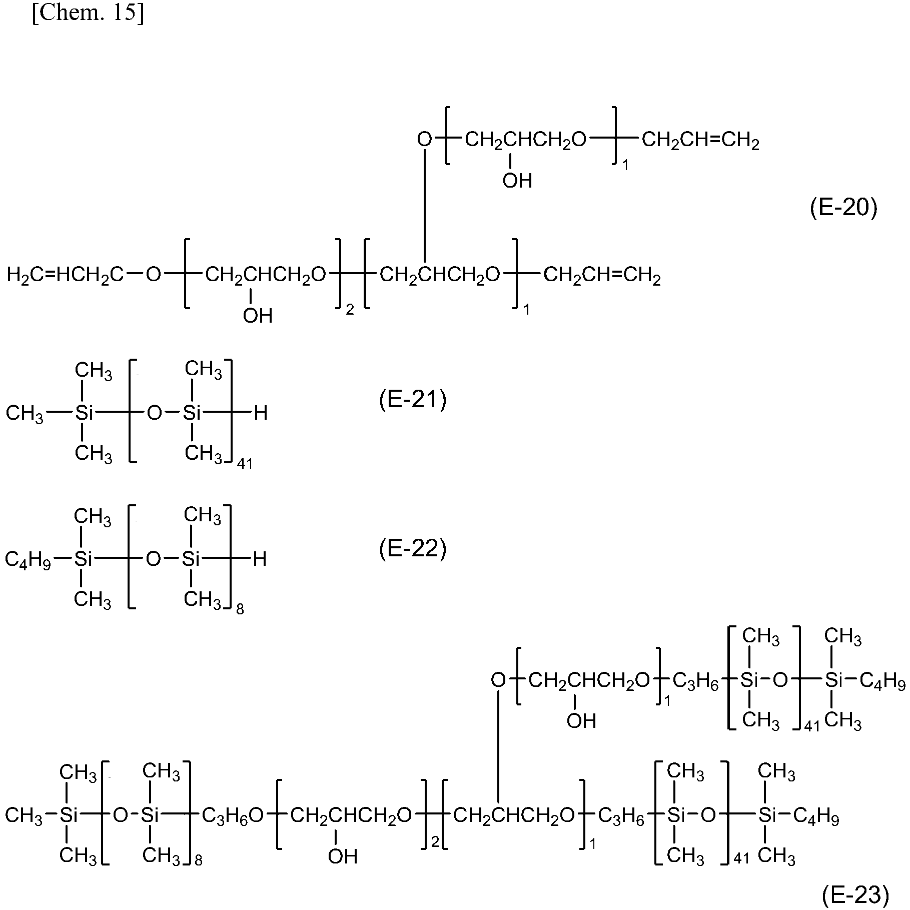

### [Example 8]

A reactor was charged with 50 g of triglycerin dioctenyl ether represented by formula (E-24) below, 1739.1 g of an organohydrogenpolysiloxane represented by formula (E-25) below (hydrosilyl groups/octenyl groups = 1.0/2.0 [by mol]), 447.3 g of isopropyl alcohol, and 0.24 g of a 3% ethanol solution of platinum-1,3-divinyl-1,1,3,3-tetramethyldisiloxane complex. The reaction was carried out by performing heating at 80°C for 5 hours. Subsequently, the reactor was charged with 52.8 g of an organohydrogenpolysiloxane represented by formula (E-26) below (hydrosilyl groups/allyl groups = 1.0/2.0 [by mol]), 13.2 g of isopropyl alcohol, and 0.01 g of a 3% ethanol solution of platinum-1,3-divinyl-1,1,3,3-tetramethyldisiloxane complex. The reaction was carried out by performing heating at 80°C for 3 hours. Subsequently, 215.0 g of 0.01 N hydrochloric acid was added and the mixture was heated at 80°C for 3 hours to perform hydrolysis. The system was then neutralized with 3.2 g of a 5% aqueous sodium hydrogencarbonate solution. The reaction solution was heated under reduced pressure to distill off the solvent, and the residue was filtered to give the target product (organopolysiloxane) as a fluid and viscous liquid represented by formula (E-27) below.

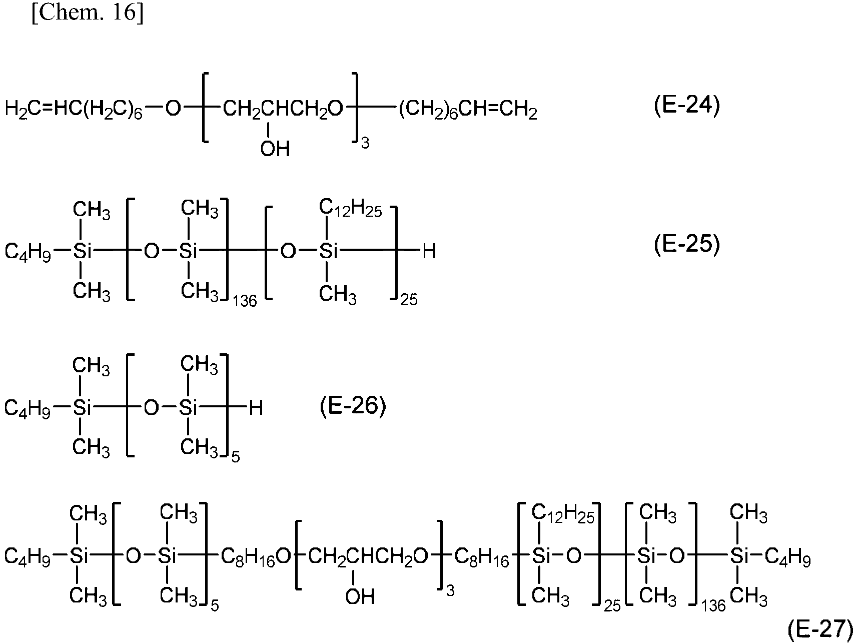

### [Example 9]

A reactor was charged with 50 g of tetraglycerin diallyl ether represented by formula (E-28) below, 710.7 g of an organohydrogenpolysiloxane represented by formula (E-29) below (hydrosilyl groups/allyl groups = 1.0/2.0 [by mol]), 532.5 g of isopropyl alcohol, and 0.15 g of a 3% ethanol solution of platinum-1,3-divinyl-1,1,3,3-tetramethyldisiloxane complex. The reaction was carried out by performing heating at 80°C for 5 hours. Subsequently, the reactor was charged with 71.1 g of an organohydrogenpolysiloxane represented by formula (E-30) below (hydrosilyl groups/allyl groups = 1.0/2.0 [by mol]), 49.8 g of isopropyl alcohol, and 0.01 g of a 3% ethanol solution of platinum-1,3-divinyl-1,1,3,3-tetramethyldisiloxane complex. The reaction was carried out by performing heating at 80°C for 3 hours. Subsequently, 93.8 g of 0.01 N hydrochloric acid was added and the mixture was heated at 80°C for 3 hours to perform hydrolysis. The system was then neutralized with 1.4 g of a 5% aqueous sodium hydrogencarbonate solution. The reaction solution was heated under reduced pressure to distill off the solvent, and the residue was filtered to give the target product (organopolysiloxane) as a fluid and viscous liquid represented by formula (E-31) below.

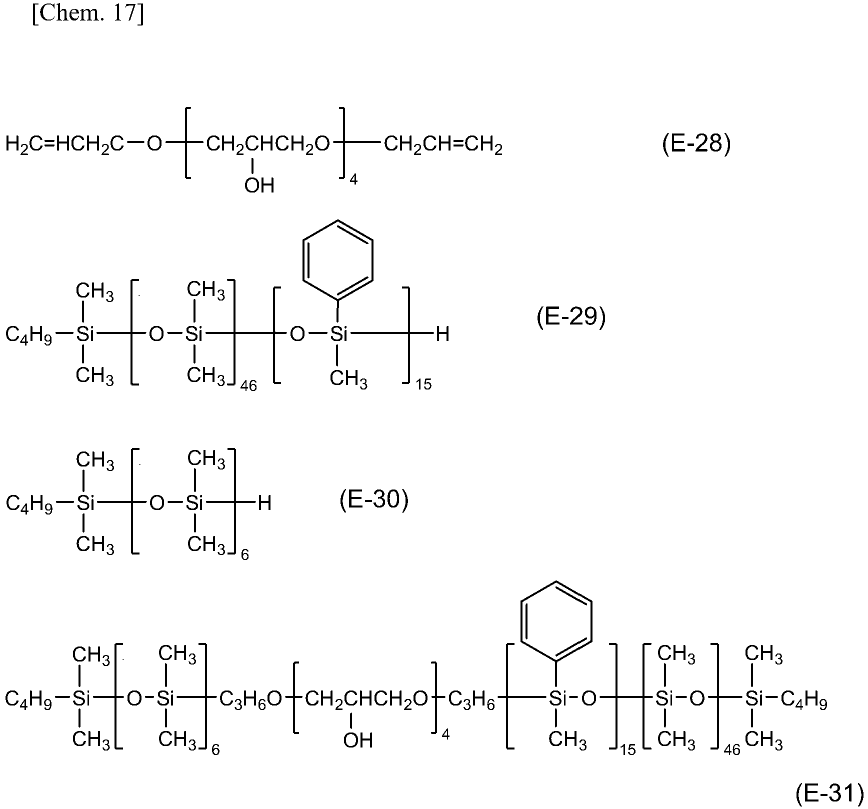

### [Example 10]

A reactor was charged with 50 g of pentaglycerin triallyl ether represented by formula (E-12), 603.4 g of an organohydrogenpolysiloxane represented by formula (E-32) below (hydrosilyl groups/allyl groups = 2.0/3.0 [by mol]), 261.4 g of isopropyl alcohol, and 0.22 g of a 3% ethanol solution of platinum-1,3-divinyl-1,1,3,3-tetramethyldisiloxane complex. The reaction was carried out by performing heating at 80°C for 5 hours. Subsequently, the reactor was charged with 56.8 g of an organohydrogenpolysiloxane represented by formula (E-33) below (hydrosilyl groups/allyl groups = 1.0/3.0 [by mol]), 22.7 g of isopropyl alcohol, and 0.02 g of a 3% ethanol solution of platinum-1,3-divinyl-1,1,3,3-tetramethyldisiloxane complex. The reaction was carried out by performing heating at 80°C for 3 hours. Subsequently, 79.2 g of 0.01 N hydrochloric acid was added and the mixture was heated at 80°C for 3 hours to perform hydrolysis. The system was then neutralized with 1.2 g of a 5% aqueous sodium hydrogencarbonate solution. The reaction solution was heated under reduced pressure to distill off the solvent, and the residue was filtered to give the target product (organopolysiloxane) as a fluid and viscous liquid represented by formula (E-34) below.

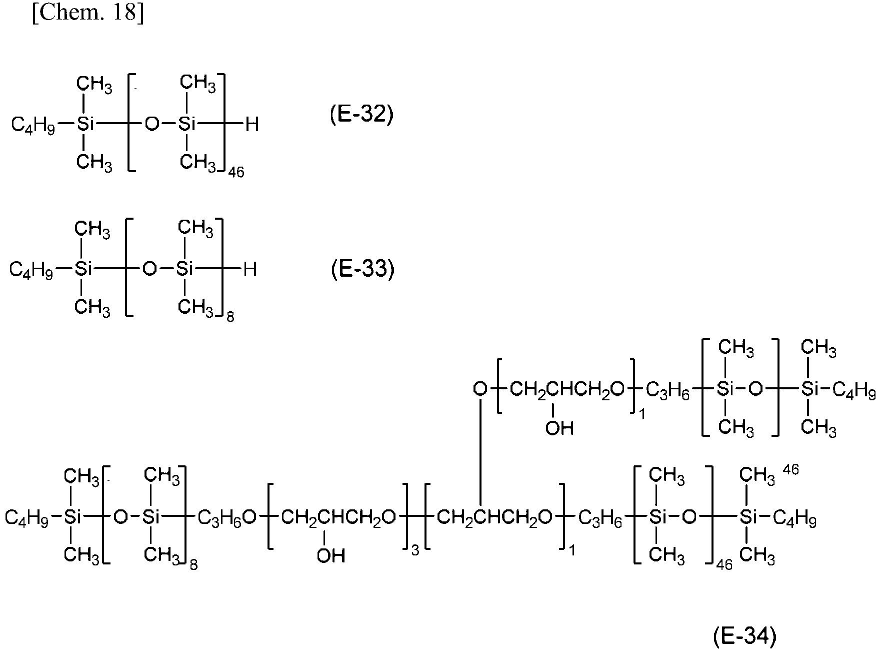

### [Comparative Example 1]

A reactor was charged with 50 g of triglycerin diallyl ether represented by formula (E-1) above, 1,060.6 g of an organohydrogenpolysiloxane represented by formula (E-35) below (hydrosilyl groups/allyl groups = 1.0/2.0 [by mol]), 333.2 g of isopropyl alcohol, and 0.19 g of a 3% ethanol solution of platinum-1,3-divinyl-1,1,3,3-tetramethyldisiloxane complex. The reaction was carried out by performing heating at 80°C for 5 hours. Subsequently, the reactor was charged with 376.3 g of an organohydrogenpolysiloxane represented by formula (E-36) below (hydrosilyl groups/allyl groups = 1.0/2.0 [by mol]), 112.9 g of isopropyl alcohol, and 0.06 g of a 3% ethanol solution of platinum-1,3-divinyl-1,1,3,3-tetramethyldisiloxane complex. The reaction was carried out by performing heating at 80°C for 3 hours. Subsequently, 172.4 g of 0.01 N hydrochloric acid was added and the mixture was heated at 80°C for 3 hours to perform hydrolysis. The system was then neutralized with 2.6 g of a 5% aqueous sodium hydrogencarbonate solution. The reaction solution was heated under reduced pressure to distill off the solvent, and the residue was filtered to give the target product (organopolysiloxane) as a fluid and viscous liquid represented by formula (E-37) below.

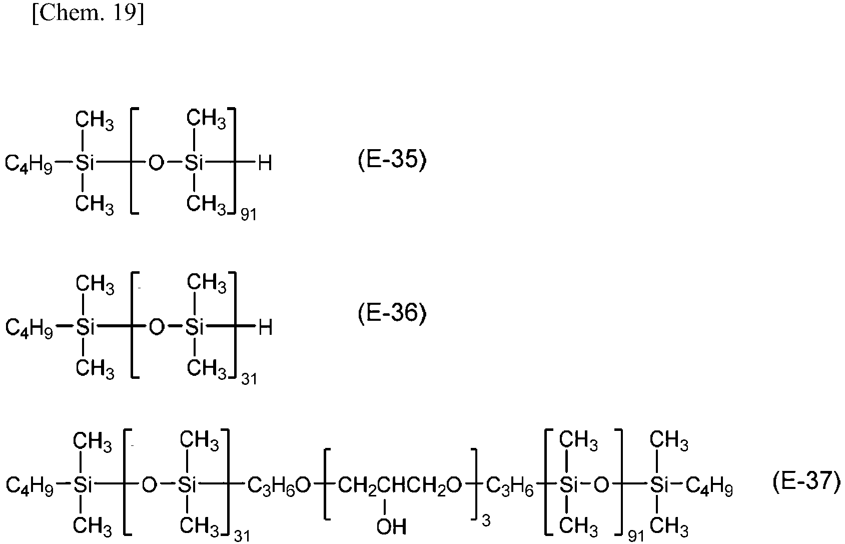

### [Comparative Example 2]

A reactor was charged with 50 g of triglycerin diallyl ether represented by formula (E-1) above, 1458.3 g of an organohydrogenpolysiloxane represented by formula (E-6) above (hydrosilyl groups/allyl groups = 2.0/2.0 [by mol]), 905.0 g of isopropyl alcohol, and 0.30 g of a 3% ethanol solution of platinum-1,3-divinyl-1,1,3,3-tetramethyldisiloxane complex. The reaction was carried out by performing heating at 80°C for 5 hours. Subsequently, 183.2 g of 0.01 N hydrochloric acid was added and the mixture was heated at 80°C for 3 hours to perform hydrolysis. The system was then neutralized with 2.8 g of a 5% aqueous sodium hydrogencarbonate solution. The reaction solution was heated under reduced pressure to distill off the solvent, and the residue was filtered to give the target product as a fluid and viscous liquid represented by formula (E-38) below.

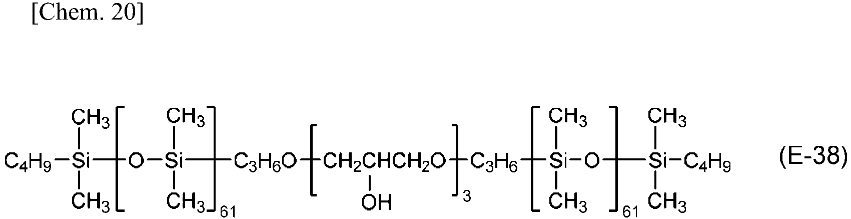

### [Comparative Example 3]

A reactor was charged with 50 g of tetraglycerin diallyl ether represented by formula (E-28) above, 1895.1 g of an organohydrogenpolysiloxane represented by formula (E-39) below (hydrosilyl groups/allyl groups = 2.0/2.0 [by mol]), 972.5 g of isopropyl alcohol, and 0.20 g of a 3% ethanol solution of platinum-1,3-divinyl-1,1,3,3-tetramethyldisiloxane complex. The reaction was carried out by performing heating at 80°C for 5 hours. Subsequently, 234.1 g of 0.01 N hydrochloric acid was added and the mixture was heated at 80°C for 3 hours to perform hydrolysis. The system was then neutralized with 3.5 g of a 5% aqueous sodium hydrogencarbonate solution. The reaction solution was heated under reduced pressure to distill off the solvent, and the residue was filtered to give the target product (organopolysiloxane) as a fluid and viscous liquid represented by formula (E-40) below.

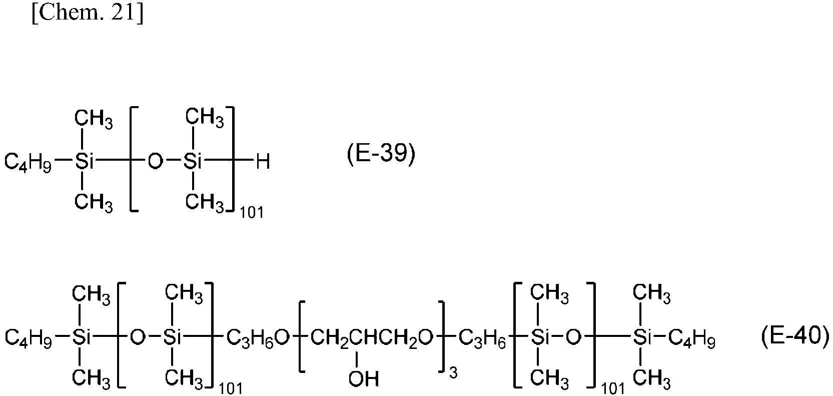

### [Comparative Example 4]

A reactor was charged with 50 g of triglycerin diallyl ether represented by formula (E-1) above, 1891.9 g of an organohydrogenpolysiloxane represented by formula (E-41) below (hydrosilyl groups/allyl groups = 2.0/2.0 [by mol]), 776.8 g of isopropyl alcohol, and 0.65 g of a 3% ethanol solution of platinum-1,3-divinyl-1,1,3,3-tetramethyldisiloxane complex. The reaction was carried out by performing heating at 80°C for 5 hours. Subsequently, 235.3 g of 0.01 N hydrochloric acid was added and the mixture was heated at 80°C for 3 hours to perform hydrolysis. The system was then neutralized with 3.5 g of a 5% aqueous sodium hydrogencarbonate solution. The reaction solution was heated under reduced pressure to distill off the solvent, and the residue was filtered to give the target product (organopolysiloxane) as a fluid and viscous liquid represented by formula (E-42) below.

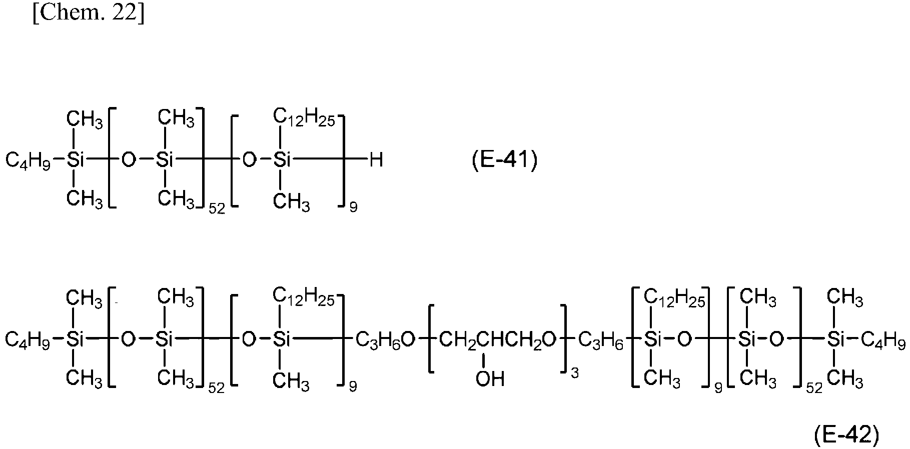

### [Comparative Example 5]

A reactor was charged with 50 g of pentaglycerin triallyl ether represented by formula (E-12), 797.6 g of an organohydrogenpolysiloxane represented by formula (E-17) above (hydrosilyl groups/allyl groups = 2.0/3.0 [by mol]), 762.9 g of isopropyl alcohol, and 0.23 g of a 3% ethanol solution of platinum-1,3-divinyl-1,1,3,3-tetramethyldisiloxane complex. The reaction was carried out by performing heating at 80°C for 5 hours. Subsequently, the reactor was charged with 110.1 g of an organohydrogenpolysiloxane represented by formula (E-43) below (hydrosilyl groups/allyl groups = 1.0/3.0 [by mol]), 99.1 g of isopropyl alcohol, and 0.03 g of a 3% ethanol solution of platinum-1,3-divinyl-1,1,3,3-tetramethyldisiloxane complex. The reaction was carried out by performing heating at 80°C for 3 hours. Subsequently, 108.9 g of 0.01 N hydrochloric acid was added and the mixture was heated at 80°C for 3 hours to perform hydrolysis. The system was then neutralized with 1.6 g of a 5% aqueous sodium hydrogencarbonate solution. The reaction solution was heated under reduced pressure to distill off the solvent, and the residue was filtered to give the target product (organopolysiloxane) as a fluid and viscous liquid represented by formula (E-44) below.

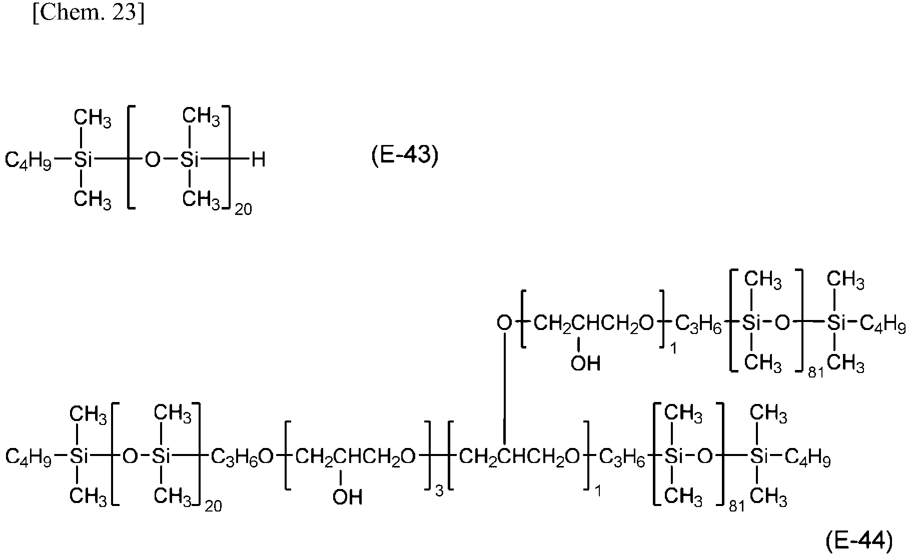

The present invention will be described in detail below by presenting exemplary formulations of Examples and Comparative Examples of cosmetic compositions. The present invention is not limited to these examples. The average particle size in the tables is the "average primary particle size".

### [Examples 11 and 12, and Comparative Examples 6 to 10]

Oily pastes were prepared according to the formulations described in Table 1.

### (Production method)

A: Ingredients (1) were dispersed using a roll to give a paste-like dispersion.

### [Processability: dispersibility]

The dispersibility was rated as "∘" when the dispersibility was good and the roll processing was easy in the step A, and was rated "×" when the dispersibility was poor and the roll processing was difficult.

### [Hardness stability]

The hardness of the paste on the day of production was compared to that after one week at 25°C. The hardness stability was rated as "⊚" when the change was less than 150%, as "○" when the change was 150% or more and less than 200%, and as poor "×" when the change was 200% or more. Those pastes that had been rated as insufficient in processability were not evaluated. The hardness was measured with Rheometer RT-2002D-D (manufactured by RHFOTECH, probe: 10 mmφ, penetration depth: 10 mm, sample stage rising speed: 5 cm/min, temperature: 25°C, range: 200).

**[Table 1]**

| Composition (parts by mass) | | Example | | Comparative Example | | | | |
|---|---|---|---|---|---|---|---|---|
| | | 11 | 12 | 6 | 7 | 8 | 9 | 10 |
| (1) | Organopolysiloxane of Example 1 | 1 | | | | | | |
| | Organopolysiloxane of Example 2 | | 1 | | | | | |
| | Organopolysiloxane of Comparative Example 1 | | | 1 | | | | |
| | Organopolysiloxane of Comparative Example 2 | | | | 1 | | | |
| | Organopolysiloxane of Comparative Example 3 | | | | | 1 | | |
| | Organopolysiloxane of Comparative Example 4 | | | | | | 1 | |
| | Organopolysiloxane of Comparative Example 5 | | | | | | | 1 |
| | Metal soap-treated microparticulate titanium oxide (Average particle size: 10 nm) | 15 | 15 | 15 | 15 | 15 | 15 | 15 |
| | KF-96A-6cs (dimethicone) | 19 | 19 | 19 | 19 | 19 | 19 | 19 |
| Total | | 35 | 35 | 35 | 35 | 35 | 35 | 35 |
| Processability | | ○ | ○ | ○ | ○ | × | × | × |
| Hardness stability | | ⊚ | ○ | × | × | - | - | - |

| | | | | | | | | |
|---|---|---|---|---|---|---|---|---|
| KF-96A-6cs: Shin-Etsu Chemical Co., Ltd. | | | | | | | | |

From the results in Table 1, the organopolysiloxanes of the present invention offer excellent dispersibility and enable production of pastes with lower viscosity. These organopolysiloxanes increased the freedom in formulation, for example, offered enhanced paste dispersibility, and also performed well in dispersion stability.

### [Example 13 and Comparative Examples 11 to 15]

Dispersions were prepared according to the formulations described in Table 2.

### (Production method)

A: Ingredients (1) were combined with zirconia beads and dispersed in a paint shaker to give a dispersion.

### [Initial viscosity]

The viscosity of the dispersion immediately after preparation was measured at 25°C. The dispersion was analyzed using a Brookfield viscometer with Spindle LV-2 at 30 rotations, and the viscosity was measured after 60 seconds. The unit is mPa·s. The initial viscosity was rated as "×" when the value was above the upper limit of measurement, 1,000 mPa·s. Viscosities that are measurable under the above conditions are low and allow for easy handling.

### [Viscosity stability]

The viscosity of the dispersion on the day of production was compared to that after one week at 25°C. The viscosity stability was rated as "^{©}" when the change was less than 150%, as "○" when the change was less than 200%, and as poor "×" when the change was 200% or more. Those dispersions that had exceeded the upper limit of viscosity measurement were not evaluated.

**[Table 2]**

| Composition (%) | | Example | Comparative Example | | | | |
|---|---|---|---|---|---|---|---|
| | | 13 | 11 | 12 | 13 | 14 | 15 |
| (1) | Organopolysiloxane of Example 3 | 5 | | | | | |
| | Organopolysiloxane of Comparative Example 1 | | 5 | | | | |
| | Organopolysiloxane of Comparative Example 2 | | | 5 | | | |
| | Organopolysiloxane of Comparative Example 3 | | | | 5 | | |
| | Organopolysiloxane of Comparative Example 4 | | | | | 5 | |
| | Organopolysiloxane of Comparative Example 5 | | | | | | 5 |
| | Metal soap-treated microparticulate titanium oxide (Average particle size: 15 nm) | 58 | 58 | 58 | 58 | 58 | 58 |
| | KF-96L-2cs (dimethicone) | 37 | 37 | 37 | 37 | 37 | 37 |
| Total | | 100 | 100 | 100 | 100 | 100 | 100 |
| Initial viscosity (mPa·s) | | 680 | × | × | × | × | × |
| Viscosity stability | | ⊚ | - | - | - | - | - |

| | | | | | | | |
|---|---|---|---|---|---|---|---|
| KF-96L-2cs (dimethicone): Shin-Etsu Chemical Co., Ltd. (The same applies hereinafter.) | | | | | | | |

From the results in Table 2, the organopolysiloxane of the present invention offers excellent dispersibility and enables production of a dispersion with lower viscosity. The organopolysiloxane increased the freedom in formulation, for example, offered enhanced dispersion dispersibility, and also performed well in dispersion stability.

### [Examples 14 and 15, and Comparative Examples 16 to 20]

Dispersions were prepared according to the formulations described in Table 3.

### (Production method)

A: Ingredients (1) were combined with zirconia beads and dispersed in a paint shaker to give a dispersion.

### [Initial viscosity]

The viscosity of the dispersion immediately after preparation was measured. The dispersion was analyzed using a Brookfield viscometer with Spindle LV-2 at 30 rotations, and the viscosity was measured after 60 seconds. The unit is mPa·s. The initial viscosity was rated as "×" when the value was above the upper limit of measurement, 1,000 mPa·s. Viscosities that are measurable under the above conditions are low and allow for easy handling.

### [Viscosity stability]

The viscosity of the dispersion on the day of production was compared to that after one week at 25°C. The viscosity stability was rated as "^{©}" when the change was less than 150%, as "○" when the change was less than 200%, and as poor "×" when the change was 200% or more. Those dispersions that had exceeded the upper limit of viscosity measurement were not evaluated.

**[Table 3]**

| Composition (%) | | Example | | Comparative Example | | | | |
|---|---|---|---|---|---|---|---|---|
| | | 14 | 15 | 16 | 17 | 18 | 19 | 20 |
| (1) | Organopolysiloxane of Example 2 | 10 | | | | | | |
| | Organopolysiloxane of Example 3 | | 10 | | | | | |
| | Organopolysiloxane of Comparative Example 1 | | | 10 | | | | |
| | Organopolysiloxane of Comparative Example 2 | | | | 10 | | | |
| | Organopolysiloxane of Comparative Example 3 | | | | | 10 | | |
| | Organopolysiloxane of Comparative Example 4 | | | | | | 10 | |
| | Organopolysiloxane of Comparative Example 5 | | | | | | | 10 |
| | Metal soap-treated microparticulate titanium oxide (Average particle size: 10 nm) | 55 | 55 | 55 | 55 | 55 | 55 | 55 |
| | KF-96L-2cs (dimethicone) | 35 | 35 | 35 | 35 | 35 | 35 | 35 |
| Total | | 100 | 100 | 100 | 100 | 100 | 100 | 100 |
| Initial viscosity (mPa·s) | | 390 | 780 | × | × | × | × | × |
| Viscosity stability | | ⊚ | ⊚ | - | - | - | - | - |

### [Example 16 and Comparative Examples 21 to 25]

Dispersions were prepared according to the formulations described in Table 4.

### (Production method)

A: Ingredients (1) were combined with zirconia beads and dispersed in a paint shaker to give a dispersion.

### [Initial viscosity]

The viscosity of the dispersion immediately after preparation was measured at 25°C. The dispersion was analyzed using a Brookfield viscometer with Spindle LV-2 at 30 rotations, and the viscosity was measured after 60 seconds. The unit is mPa·s. The initial viscosity was rated as "×" when the value was above the upper limit of measurement, 1,000 mPa·s. Viscosities that are measurable under the above conditions are low and allow for easy handling.

### [Viscosity stability]

The viscosity of the dispersion on the day of production was compared to that after one week at 25°C. The viscosity stability was rated as "⊚" when the change was less than 150%, as "○" when the change was less than 200%, and as poor "×" when the change was 200% or more. Those dispersions that had exceeded the upper limit of viscosity measurement were not evaluated.

**[Table 4]**

| Composition (%) | | Example | Comparative Example | | | | |
|---|---|---|---|---|---|---|---|
| | | 16 | 21 | 22 | 23 | 24 | 25 |
| (1) | Organopolysiloxane of Example 3 | 5 | | | | | |
| | Organopolysiloxane of Comparative Example 1 | | 5 | | | | |
| | Organopolysiloxane of Comparative Example 2 | | | 5 | | | |
| | Organopolysiloxane of Comparative Example 3 | | | | 5 | | |
| | Organopolysiloxane of Comparative Example 4 | | | | | 5 | |
| | Organopolysiloxane of Comparative Example 5 | | | | | | 5 |
| | Metal soap-treated microparticulate zinc oxide (Average particle size: 35 nm) | 68 | 68 | 68 | 68 | 68 | 68 |
| | KF-96L-2cs (dimethicone) | 27 | 27 | 27 | 27 | 27 | 27 |
| Total | | 100 | 100 | 100 | 100 | 100 | 100 |
| Initial viscosity (mPa·s) | | 510 | × | × | × | × | × |
| Viscosity stability | | ⊚ | - | - | - | - | - |

From the results in Table 4, the organopolysiloxane of the present invention offers excellent dispersibility and enables production of a dispersion with lower viscosity. The organopolysiloxane increases the freedom in formulation, for example, offers enhanced dispersion dispersibility, and preferred viscosity and powder dispersion stability are obtained.

### [Examples 17 to 20, and Comparative Examples 26 to 30]

Emulsion cosmetic compositions were prepared according to the formulations described in Table 5.

### (Production method)

A: Ingredients (2) were added to ingredients (1) and emulsified to give a cream.

### [Feeling on use]

The sample (the cream) was applied to the cheeks of a panel of 10 experts and the stickiness of the cream upon application was sensorily evaluated. The evaluation criteria were 5 points when there was almost no stickiness, 3 points when the cream was slightly sticky, and 1 point when stickiness was felt. The scores were combined.
⊚: Total score is 45 points or more.
○: Total score is 40-44 points.
Δ: Total score is 21-39 points.
×: Total score is 20 points or less.

Those samples that had "○" or higher rating were accepted.

### [Spreadability]

The sample (the cream) was applied to the cheeks of a panel of 10 experts and the spreadability of the cream upon application was sensorily evaluated. The evaluation criteria were 5 points when the spreadability was good, 3 points when the spreadability was slightly poor, and 1 point when the spreadability was low. The scores were combined.
⊚: Total score is 45 points or more.
○: Total score is 40-44 points.
△: Total score is 21-39 points.
×: Total score is 20 points or less.

Those samples that had "○" or higher rating were accepted.

**[Table 5]**

| Composition (%) | | Example | | | | Comparative Example | | | | |
|---|---|---|---|---|---|---|---|---|---|---|
| | | 17 | 18 | 19 | 20 | 26 | 27 | 28 | 29 | 30 |
| (1) | Organopolysiloxane of Example 1 | 3.0 | | | | | | | | |
| | Organopolysiloxane of Example 2 | | 3.0 | | | | | | | |
| | Organopolysiloxane of Example 3 | | | 3.0 | | | | | | |
| | Organopolysiloxane of Example 4 | | | | 3.0 | | | | | |
| | Organopolysiloxane of Comparative Example 1 | | | | | 3.0 | | | | |
| | Organopolysiloxane of Comparative Example 2 | | | | | | 3.0 | | | |
| | Organopolysiloxane of Comparative Example 3 | | | | | | | 3.0 | | |
| | Organopolysiloxane of Comparative Example 4 | | | | | | | | 3.0 | |
| | Organopolysiloxane of Comparative Example 5 | | | | | | | | | 3.0 |
| | Isononyl isononanoate | 6.0 | 6.0 | 6.0 | 6.0 | 6.0 | 6.0 | 6.0 | 6.0 | 6.0 |
| | KF-96A-2cs (dimethicone) | 6.0 | 6.0 | 6.0 | 6.0 | 6.0 | 6.0 | 6.0 | 6.0 | 6.0 |
| (2) | BG | 5.0 | 5.0 | 5.0 | 5.0 | 5.0 | 5.0 | 5.0 | 5.0 | 5.0 |
| | Sodium chloride | 0.5 | 0.5 | 0.5 | 0.5 | 0.5 | 0.5 | 0.5 | 0.5 | 0.5 |
| | Water | 79.5 | 79.5 | 79.5 | 79.5 | 79.5 | 79.5 | 79.5 | 79.5 | 79.5 |
| Total | | 100.0 | 100.0 | 100.0 | 100.0 | 100.0 | 100.0 | 100.0 | 100.0 | 100.0 |
| Feeling on use | | ⊚ | ⊚ | ⊚ | ○ | ○ | ○ | ○ | Δ | Δ |
| Spreadability | | ○ | ○ | ⊚ | ⊚ | △ | △ | △ | × | × |

From the results in Table 5, the organopolysiloxanes of the present invention were shown to have excellent feeling on use and spreadability.

### [Example 21] O/W sunscreen cream

| | Composition | % |
|---|---|---|
| 1. | Organopolysiloxane of Example 3 | 0.5 |
| 2. | Silicone-treated microparticulate zinc oxide | 5 |
| 3. | Decamethylcyclopentasiloxane | 5 |
| 4. | Ethylhexyl methoxycinnamate | 5 |
| 5. | KF-56A (Note 1) | 3 |
| 6. | Cetanol | 0.5 |
| 7. | Polyoxyethylene sorbitan monooleate | 2.5 |
| 8. | Glyceryl stearate (SE) | 0.5 |
| 9. | KF-6011P (Note 2) | 1 |
| 10. | Dipropylene glycol | 6 |
| 11. | 1,3-Butylene glycol | 6 |
| 12. | Carboxyvinyl polymer | 0.3 |
| 13. | Acrylic polymer compound | 0.3 |
| 14. | Methylparaben | 0.2 |
| 15. | Phenoxyethanol | 0.3 |
| 16. | Disodium edetate | q.s. |
| 17. | Water | Balance |
| 18. | 10% Aqueous sodium hydroxide solution | q.s. |
| | Total | 100.0 |

| | | |
|---|---|---|
| (Note 1) Diphenylsiloxyphenyl trimethicone, Shin-Etsu Chemical Co., Ltd. (Note 2) PEG-11 methyl ether dimethicone, Shin-Etsu Chemical Co., Ltd. | | |

### (Production method)

A: Ingredients 1 to 3 were mixed uniformly in a bead mill.
B: The mixture obtained in A and ingredients 4 to 6 were mixed uniformly at 90°C.
C: Ingredients 7 to 17 were mixed uniformly at 85°C.
D: The mixture obtained in B was added to the mixture obtained in C and emulsified, and ingredient 18 was added and mixed uniformly to give an O/W sunscreen.

The O/W sunscreen obtained had high transparency, was low in stickiness, and had a good feeling on use.

### [Example 22] W/O cream

| | Composition | % |
|---|---|---|
| 1. | KSG-710 (Note 1) | 4 |
| 2. | KSG-15 (Note 2) | 1 |
| 3. | Organopolysiloxane of Example 5 | 3 |
| 4. | Dimethylpolysiloxane (6 cs) | 13 |
| 5. | 1,3-Butylene glycol | 8 |
| 6. | Sorbitol | 5 |
| 7. | Phenoxyethanol | 0.3 |
| 8. | Sodium citrate | q.s. |
| 9. | Sodium chloride | q.s. |
| 10. | Water | Balance |
| | Total | 100.0 |

| | | |
|---|---|---|
| (Note 1) Mixture of 70-80% dimethicone + 20-30% (dimethicone/polyglycerin-3) crosspolymer, Shin-Etsu Chemical Co., Ltd. (Note 2) Mixture of 90-96% cyclopentasiloxane + 4-10% (dimethicone/vinyl dimethicone) crosspolymer, Shin-Etsu Chemical Co., Ltd. | | |

### (Production method)

A: Ingredients 1 to 4 were mixed uniformly.
B: Ingredients 5 to 10 were mixed uniformly.
C: The mixture obtained in B was added to the mixture obtained in A and emulsified to give a W/O cream.

The W/O cream obtained had a moisturizing feel, was not sticky, and had a good feeling on use.

### [Example 23] W/O shaking sunscreen

| | Composition | % |
|---|---|---|
| 1. | Organopolysiloxane of Example 1 | 2.8 |
| 2. | Organopolysiloxane of Example 6 | 1.7 |
| 3. | Metal soap-treated microparticulate titanium oxide | 14 |
| 4. | Hydrogen dimethicone-treated microparticulate zinc oxide | 21 |
| 5. | Decamethylcyclopentasiloxane | 12 |
| 6. | Decamethylcyclopentasiloxane | 12 |
| 7. | KSG-15 (Note 1) | 2 |
| 8. | KSG-16 (Note 2) | 4 |
| 9. | KF-6104 (Note 3) | 0.5 |
| 10. | Decamethylcyclopentasiloxane | Balance |
| 11. | Dimethylpolysiloxane (6 cs) | 3 |
| 12. | Isotridecyl isononanoate | 4.8 |
| 13. | KSP-105 (Note 4) | 1 |
| 14. | 1,3-Butylene glycol | 2 |
| 15. | Phenoxyethanol | q.s. |
| 16. | Sodium citrate | q.s. |
| 17. | Sodium chloride | q.s. |
| 18. | Purified water | 13 |
| | Total | 100.0 |

| | | |
|---|---|---|
| (Note 1) Mixture of 90-96% cyclopentasiloxane + 4-10% (dimethicone/vinyl dimethicone) crosspolymer, Shin-Etsu Chemical Co., Ltd. (Note 2) Mixture of 70-80% dimethicone + 20-30% (dimethicone/vinyl dimethicone) crosspolymer, Shin-Etsu Chemical Co., Ltd. (Note 3) Polyglyceryl-3 polydimethylsiloxyethyl dimethicone, Shin-Etsu Chemical Co., Ltd. (Note 4) (Vinyl dimethicone/methicone silsesquioxane) crosspolymer, Shin-Etsu Chemical Co., Ltd. | | |

### (Production method)

A: Ingredients 1, 3, and 5 were mixed uniformly using a homomixer.
B: Ingredients 2, 4, and 6 were mixed uniformly using a homomixer.
C: The mixture obtained in A, the mixture obtained in B, and ingredients 7 to 12 were mixed uniformly.
D: Ingredients 13 to 18 were mixed uniformly.
E: The mixture obtained in D was added to the mixture obtained in C and emulsified to give a W/O shaking sunscreen.

The W/O shaking sunscreen obtained, in spite of being highly loaded with microparticulate powder, had low viscosity, high transparency, no stickiness, and good feeling on use. Furthermore, the sunscreen had no or a minor increase in viscosity at high temperatures and was excellent in stability.

### [Example 24] W/O liquid foundation

| | Composition | % |
|---|---|---|
| 1. | KSG-210 (Note 1) | 3 |
| 2. | KSG-44 (Note 2) | 7 |
| 3. | KF-6028 (Note 3) | 2 |
| 4. | KF-6038 (Note 4) | 1 |
| 5. | Trisiloxane | Balance |
| 6. | Organically modified clay mineral | 1 |
| 7. | Organopolysiloxane of Example 7 | 1 |
| 8. | Dimethylpolysiloxane (6 cs) | 5 |
| 9. | Isotridecyl isononanoate | 7 |
| 10. | KTP-09W (Note 5) | 8.5 |
| 11. | KTP-09Y (Note 5) | 1 |
| 12. | KTP-09R (Note 5) | 0.4 |
| 13. | KTP-09B (Note 5) | 0.1 |
| 14. | Metal soap-treated microparticulate titanium oxide | 5 |
| 15. | Dipropylene glycol | 5 |
| 16. | Phenoxyethanol | 0.2 |
| 17. | Sodium citrate | q.s. |
| 18. | Magnesium sulfate | q.s. |
| 19. | Water | 33 |
| | Total | 100.0 |

| | | |
|---|---|---|
| (Note 1) Mixture of 70-80% dimethicone + 20-30% (dimethicone/(PEG-10/15)) crosspolymer, Shin-Etsu Chemical Co., Ltd. (Note 2) Mixture of 65-75% squalane + 25-35% (vinyl dimethicone/lauryl dimethicone) crosspolymer, Shin-Etsu Chemical Co., Ltd. (Note 3) PEG-9 polydimethylsiloxyethyl dimethicone, Shin-Etsu Chemical Co., Ltd. (Note 4) Lauryl PEG-9 polydimethylsiloxyethyl dimethicone, Shin-Etsu Chemical Co., Ltd. (Note 5) KF-9909-treated coloring inorganic pigments, W: white, R: red, Y: yellow, B: black, Shin-Etsu Chemical Co., Ltd. | | |

### (Production method)

A: Ingredients 7 to 14 were mixed together using a triple roll mill to give a paste.
B: The mixture obtained in A and ingredients 1 to 6 were mixed uniformly.
C: Ingredients 15 to 19 were mixed uniformly.
D: The mixture obtained in C was added to the mixture obtained in B and emulsified to give a W/O liquid foundation.

The W/O liquid foundation obtained, in spite of being highly loaded with powder, had low viscosity, good spreadability, and a good feeling on use. Furthermore, the liquid foundation had no or a minor increase in viscosity at high temperatures and was excellent in stability.

### [Example 25] W/O cream foundation

| Composition | | % |
|---|---|---|
| 1. | KSG-270 (Note 1) | 3 |
| 2. | KSG-18A (Note 2) | 7 |
| 3. | KF-6038 (Note 3) | 3 |
| 4. | Hydrogenated farnesene | Balance |
| 5. | Ethylhexyl triazone | 4 |
| 6. | Diethylamino hydroxybenzoyl hexyl benzoate | 1 |
| 7. | Organically modified clay mineral | 1.2 |
| 8. | Organopolysiloxane of Example 8 | 1.5 |
| 9. | KF-56A (Note 4) | 10 |
| 10. | Coco-alkyl (caprylate/caprate) | 7 |
| 11. | KTP-09W (Note 5) | 8.5 |
| 12. | KTP-09Y (Note 5) | 1 |
| 13. | KTP-09R (Note 5) | 0.4 |
| 14. | KTP-09B (Note 5) | 0.1 |
| 15. | Metal soap-treated microparticulate titanium oxide | 10 |
| 16. | Dipropylene glycol | 5 |
| 17. | Phenoxyethanol | 0.2 |
| 18. | Sodium citrate | q.s. |
| 19. | Magnesium sulfate | q.s. |
| 20. | Purified water | 30 |
| Total | | 100.0 |

| | | |
|---|---|---|
| (Note 1) Mixture of 75-85% diphenylsiloxyphenyl trimethicone + 15-25% (dimethicone/(PEG-10/15)) crosspolymer, Shin-Etsu Chemical Co., Ltd. (Note 2) Mixture of 80-90% diphenylsiloxyphenyl trimethicone + 10-20% (dimethicone/phenyl vinyl dimethicone) crosspolymer, Shin-Etsu Chemical Co., Ltd. (Note 3) Lauryl PEG-9 polydimethylsiloxyethyl dimethicone, Shin-Etsu Chemical Co., Ltd. (Note 4) Diphenylsiloxyphenyl trimethicone, Shin-Etsu Chemical Co., Ltd. (Note 5) KF-9909-treated coloring inorganic pigments, W: white, R: red, Y: yellow, B: black, Shin-Etsu Chemical Co., Ltd. | | |

### (Production method)

A: Ingredients 8 to 15 were mixed together using a triple roll mill to give a paste.
B: The mixture obtained in A and ingredients 1 to 7 were mixed uniformly.
C: Ingredients 16 to 20 were mixed uniformly.
D: The mixture obtained in C was added to the mixture obtained in B and emulsified to give a W/O cream foundation.

The W/O cream foundation obtained, in spite of being highly loaded with powder, had good spreadability and a good feeling on use.

### [Example 26] Non-aqueous concealer

| Composition | | % |
|---|---|---|
| 1. | KSP-101 (Note 1) | 23 |
| 2. | KSP-300 (Note 2) | 5 |
| 3. | KSG-19 (Note 3) | 6 |
| 4. | Dimethylpolysiloxane (6 cs) | 40 |
| 5. | KF-56A (Note 4) | 7 |
| 6. | TMF-1.5 (Note 5) | Balance |
| 7. | Triethylhexanoin | 0.2 |
| 8. | Organopolysiloxane of Example 9 | 0.5 |
| 9. | AES-3083-treated titanium oxide (Note 6) | 0.3 |
| 10. | AES-3083-treated iron oxide (Note 6) | q.s. |
| Total | | 100.0 |

| | | |
|---|---|---|
| (Note 1) (Vinyl dimethicone/methicone silsesquioxane) crosspolymer, Shin-Etsu Chemical Co., Ltd. (Note 2) (Diphenyl dimethicone/vinyl diphenyl dimethicone/silsesquioxane) crosspolymer, Shin-Etsu Chemical Co., Ltd. (Note 3) Mixture of 80-90% dimethicone + 10-20% (dimethicone/vinyl dimethicone) crosspolymer, Shin-Etsu Chemical Co., Ltd. (Note 4) Diphenylsiloxyphenyl trimethicone, Shin-Etsu Chemical Co., Ltd. (Note 5) Methyl trimethicone, Shin-Etsu Chemical Co., Ltd. (Note 6) Triethoxycaprylylsilane-treated coloring inorganic pigment, Shin-Etsu Chemical Co., Ltd. | | |

### (Production method)

A: Ingredients 7 to 10 were mixed together using a dispersing mixer to give a paste.
B: The mixture obtained in A and ingredients 1 to 6 were mixed uniformly to give a non-aqueous concealer.

The non-aqueous concealer obtained had good powder dispersibility and a good feeling on use.

### [Example 27] Lipstick

| Composition | | % |
|---|---|---|
| 1. | Candelilla wax | 3 |
| 2. | Polyethylene | 2 |
| 3. | Microcrystalline wax | 2.5 |
| 4. | Ceresin | 6 |
| 5. | KP-561P (Note 1) | 13 |
| 6. | Macadamia nut oil | 23 |
| 7. | Diisostearyl malate | 8.5 |
| 8. | Hydrogenated polyisobutene | 8.5 |
| 9. | Isotridecyl isononanoate | 15 |
| 10. | Polyglyceryl-2 triisostearate | 4.5 |
| 11. | Organopolysiloxane of Example 10 | 2.5 |
| 12. | Red #201 | 0.4 |
| 13. | Red #202 | 0.4 |
| 14. | Yellow #4 | 1.6 |
| 15. | KTP-09W (Note 2) | 3 |
| 16. | KTP-09R (Note 2) | 0.7 |
| 17. | KTP-09B (Note 2) | 0.2 |
| 18. | KF-9909-treated (Note 3) talc | 0.7 |
| 19. | Mica | 4.5 |
| Total | | 100.0 |

| | | |
|---|---|---|
| (Note 1) (Acrylates/stearyl acrylate/dimethicone methacrylate) copolymer, Shin-Etsu Chemical Co., Ltd. (Note 2) KF-9909-treated coloring inorganic pigments, W: white, R: red, B: black, Shin-Etsu Chemical Co., Ltd. (Note 3) Triethoxysilylethyl polydimethylsiloxyethylhexyl dimethicone, Shin-Etsu Chemical Co., Ltd. | | |

### (Production method)

A: Ingredients 10 to 18 were dispersed using a roll mill.
B: Ingredients 1 to 9 were mixed uniformly at 90°C.
C: The mixture obtained in A and ingredient 19 were added to the mixture obtained in B. The resultant mixture was mixed uniformly at 80°C, poured into a mold, and then slowly cooled to give a lipstick.

The lipstick obtained had excellent adhesion and a good feeling on use. This shows that the organopolysiloxane can be applied to other non-aqueous oily cosmetic compositions, such as glosses, stick concealers, and solid foundations.

### [Example 28] W/O solid foundation

| Composition | | % |
|---|---|---|
| 1. | KSG-710 (Note 1) | 5 |
| 2. | KSG-19 (Note 2) | 3 |
| 3. | Organopolysiloxane of Example 3 | 2 |
| 4. | SPD-Z7L (Note 3) | 10 |
| 5. | KF-56A (Note 4) | 7.35 |
| 6. | Ceresin | 5 |
| 7. | Octyldodecyl myristate | 2 |
| 8. | KF-9901-treated titanium oxide (Note 5) | 7 |
| 9. | KF-9901-treated black iron oxide (Note 5) | 0.1 |
| 10. | KF-9901-treated red iron oxide (Note 5) | 0.3 |
| 11. | KF-9901-treated yellow iron oxide (Note 5) | 0.6 |
| 12. | KP-549 (Note 6) | 0.3 |
| 13. | BG | 7 |
| 14. | Glycerin | 5 |
| 15. | Sodium citrate | 0.2 |
| 16. | Sodium chloride | 1 |
| 17. | Water | Balance |
| Total | | 100.0 |

| | | |
|---|---|---|
| (Note 1) Mixture of 70-80% dimethicone (6 cs) + 20-30% (dimethicone/polyglycerin-3) crosspolymer, Shin-Etsu Chemical Co., Ltd. (Note 2) Mixture of 80-90% dimethicone (6 cs) + 10-20% (dimethicone/vinyl dimethicone) crosspolymer, Shin-Etsu Chemical Co., Ltd. (Note 3) Dimethicone (2 cs) dispersion of 60% microparticulate zinc oxide, Shin-Etsu Chemical Co., Ltd. (Note 4) Diphenylsiloxyphenyl trimethicone, Shin-Etsu Chemical Co., Ltd. (Note 5) Hydrogen dimethicone-treated coloring inorganic pigment, Shin-Etsu Chemical Co., Ltd. (Note 6) (Acrylates/dimethicone) copolymer, Shin-Etsu Chemical Co., Ltd. | | |

### (Production method)

A: Ingredients 7 to 12 were dispersed using a high-pressure disperser.
B: Ingredients 1 to 6 were dissolved at 90°C, and the mixture obtained in A was added and mixed uniformly.
C: Ingredients 13 to 17 were mixed uniformly.
D: The mixture obtained in C was added to the mixture obtained in B and emulsified to give a W/O solid foundation.

The W/O solid foundation obtained had a good feeling on use, good applicability, and excellent storage stability.

### [Example 29] Lipstick

| Composition | | % |
|---|---|---|
| 1. | Synthetic wax | 6 |
| 2. | Paraffin | 6 |
| 3. | Microcrystalline wax | 2 |
| 4. | Organopolysiloxane of Example 9 | 2.5 |
| 5. | KF-56A (Note 1) | 5 |
| 6. | Dipentaerythrityl tripolyhydroxystearate | Balance |
| 7. | Jojoba seed oil | 1.5 |
| 8. | Hydrogenated polyisobutene | 12 |
| 9. | Di(phytosteryl/octyldodecyl) lauroyl glutamate | 1.5 |
| 10. | Organopolysiloxane of Example 10 | 3 |
| 11. | Triethylhexanoin | 15 |
| 12. | Glyceryl tri(caprylate/caprate) | 7 |
| 13. | Red #202 | 0.6 |
| 14. | Yellow #4 | 2.2 |
| 15. | KTP-09W (Note 2) | 5 |
| 16. | KF-9909-treated (Note 3) mica | 2.5 |
| 17. | Red #201 | 0.4 |
| 18. | Isotridecyl isononanoate | 4.5 |
| 19. | Polyglyceryl-2 triisostearate | 3.0 |
| Total | | **100.0** |

| | | |
|---|---|---|
| (Note 1) Diphenylsiloxyphenyl trimethicone, Shin-Etsu Chemical Co., Ltd. (Note 2) KF-9909-treated coloring inorganic pigments, W: white, R: red, B: black, Shin-Etsu Chemical Co., Ltd. (Note 3) Triethoxysilylethyl polydimethylsiloxyethylhexyl dimethicone, Shin-Etsu Chemical Co., Ltd. | | |

### (Production method)

A: Ingredients 13 to 19 were dispersed using a roll mill.
B: Ingredients 1 to 12 were mixed uniformly at 90°C.
C: The mixture obtained in A and ingredient 19 were added to the mixture obtained in B. The resultant mixture was mixed uniformly at 80°C, poured into a mold, and then slowly cooled to give a lipstick.

The lipstick obtained had excellent adhesion, transfer resistance, and gloss, and also had a good feeling on use.

### [Example 30] W/O sunscreen cream

| Composition | | % |
|---|---|---|
| 1. | KSG-820 (Note 1) | 3.5 |
| 2. | KSG-42A (Note 2) | 3 |
| 3. | KF-6105 (Note 3) | 3 |
| 4. | KF-4422 (Note 4) | Balance |
| 5. | Disteardimonium hectorite | 1 |
| 6. | Diethylamino hydroxybenzoyl hexyl benzoate | 3 |
| 7. | Bis-ethylhexyloxyphenol methoxyphenyl triazine | 2 |
| 8. | Ethylhexyl triazone | 4 |
| 9. | KF-56A (Note 5) | 5 |
| 10. | Dispersion of Example 14 | 15 |
| 11. | Dispersion of Example 16 | 15 |
| 12. | KSP-300 (Note 6) | 1.5 |
| 13. | BG | 3 |
| 14. | Ethanol | 5 |
| 15. | Ethylhexylglycerin | q.s. |
| 16. | Sodium citrate | q.s. |
| 17. | Sodium chloride | q.s. |
| 18. | Purified water | 30 |
| Total | | 100.0 |

| | | |
|---|---|---|
| (Note 1) Mixture of 70-80% isododecane + 20-30% (dimethicone/polyglyceryl-3) crosspolymer, Shin-Etsu Chemical Co., Ltd. (Note 2) Mixture of 75-85% isododecane + 15-25% (vinyl dimethicone/lauryl dimethicone) crosspolymer, Shin-Etsu Chemical Co., Ltd. (Note 3) Lauryl polyglyceryl-3 polydimethylsiloxyethyl dimethicone, Shin-Etsu Chemical Co., Ltd. (Note 4) Ethyl methicone, Shin-Etsu Chemical Co., Ltd. (Note 5) Diphenylsiloxyphenyl trimethicone, Shin-Etsu Chemical Co., Ltd. (Note 6) (Diphenyl dimethicone/vinyl diphenyl dimethicone/silsesquioxane) crosspolymer, Shin-Etsu Chemical Co., Ltd. | | |

### (Production method)

A: Ingredients 1 to 9 were mixed uniformly.
B: Ingredients 13 to 18 were mixed uniformly.
C: The mixture obtained in B was added to the mixture obtained in A and emulsified, and ingredients 10 to 12 were added thereto to give a W/O sunscreen.

The W/O sunscreen obtained, in spite of being highly loaded with fine powder, had low viscosity, high transparency, no stickiness, and good feeling on use. Furthermore, the sunscreen had no or a minor increase in viscosity at high temperatures and was excellent in stability.

### [Examples 31 to 37]

Oily paste dispersions were prepared according to the formulations described in Table 6.

### (Production method)

Ingredients (1) were dispersed using a roll to give an oily paste dispersion.

**[Table 6]**

| Composition (%) | | Example | | | | | | |
|---|---|---|---|---|---|---|---|---|
| | | 31 | 32 | 33 | 34 | 35 | 36 | 37 |
| (1) | Organopolysiloxane of Example 1 | 4.0 | | | | | | |
| | Organopolysiloxane of Example 2 | | 4.0 | | 4.0 | 4.0 | 4.0 | 4.0 |
| | Organopolysiloxane of Example 3 | | | 4.0 | | | | |
| | KF-96A-6cs (dimethicone) | 15.0 | 15.0 | 15.0 | | | | |
| | KF-4418 (Note 1) | | | | 15.0 | | | |
| | KF-56A (Note 2) | | | | | 15.0 | | |
| | Coco-alkyl (caprylate/caprate) | | | | | | 15.0 | |
| | (C13-15) alkane | | | | | | | 15.0 |
| | KTP-09W (Note 3) | 72.6 | 72.6 | 72.6 | 72.6 | 72.6 | 72.6 | 72.6 |
| | KTP-09Y (Note 3) | 6.0 | 6.0 | 6.0 | 6.0 | 6.0 | 6.0 | 6.0 |
| | KTP-09R (Note 3) | 1.6 | 1.6 | 1.6 | 1.6 | 1.6 | 1.6 | 1.6 |
| | KTP-09B (Note 3) | 0.8 | 0.8 | 0.8 | 0.8 | 0.8 | 0.8 | 0.8 |
| Total | | 100.0 | 100.0 | 100.0 | 100.0 | 100.0 | 100.0 | 100.0 |

| | | | | | | | | |
|---|---|---|---|---|---|---|---|---|
| (Note 1) Caprylyl methicone, Shin-Etsu Chemical Co., Ltd. (Note 2) Diphenylsiloxyphenyl trimethicone, Shin-Etsu Chemical Co., Ltd. (Note 3) KF-9909-treated coloring inorganic pigments, W: white, R: red, Y: yellow, B: black, Shin-Etsu Chemical Co., Ltd. | | | | | | | | |

### [Examples 38 to 44]

Oily paste dispersions were prepared according to the formulations described in Table 7.

### (Production method)

Ingredients (1) were dispersed using a roll to give an oily paste dispersion.

**[Table 7]**

| Composition (%) | | Example | | | | | | |
|---|---|---|---|---|---|---|---|---|
| | | 38 | 39 | 40 | 41 | 42 | 43 | 44 |
| (1) | Organopolysiloxane of Example 3 | 3.2 | 3.2 | 3.2 | 3.2 | 3.2 | 3.2 | 3.2 |
| | KF-96A-6cs (dimethicone) | 14.6 | 14.6 | 14.6 | | | | |
| | KF-4418 (Note 1) | | | | 14.6 | | | |
| | KF-56A (Note 2) | | | | | 14.6 | | |
| | Coco-alkyl (caprylate/caprate) | | | | | | 14.6 | |
| | (C13-15) alkane | | | | | | | 14.6 |
| | KTP-09W (Note 3) | 52.8 | 52.8 | 52.8 | 52.8 | 52.8 | 52.8 | 52.8 |
| | KTP-09Y (Note 3) | 5.9 | 5.9 | 5.9 | 5.9 | 5.9 | 5.9 | 5.9 |
| | KTP-09R (Note 3) | 1.1 | 1.1 | 1.1 | 1.1 | 1.1 | 1.1 | 1.1 |
| | KTP-09B (Note 3) | 0.4 | 0.4 | 0.4 | 0.4 | 0.4 | 0.4 | 0.4 |
| | Silicone-treated talc (Note 3) | 22.0 | | 18.0 | 18.0 | 18.0 | 18.0 | 18.0 |
| | Barium sulfate | | 22.0 | 4.0 | 4.0 | 4.0 | 4.0 | 4.0 |
| Total | | 100.0 | 100.0 | 100.0 | 100.0 | 100.0 | 100.0 | 100.0 |

| | | | | | | | | |
|---|---|---|---|---|---|---|---|---|
| (Note 1) Caprylyl methicone, Shin-Etsu Chemical Co., Ltd. (Note 2) Diphenylsiloxyphenyl trimethicone, Shin-Etsu Chemical Co., Ltd. (Note 3) KF-9909-treated coloring inorganic pigments, W: white, R: red, Y: yellow, B: black, KF-9909-treated talc, Shin-Etsu Chemical Co., Ltd. | | | | | | | | |

### [Example 45] W/O liquid foundation

| Composition | | % |
|---|---|---|
| 1. | KSG-710 (Note 1) | 4 |
| 2. | KSG-43 (Note 2) | 5 |
| 3. | KF-6105 (Note 3) | 4 |
| 4. | TMF-1.5 (Note 4) | Balance |
| 5. | Organically modified clay mineral | 1 |
| 6. | Isononyl isononanoate | 1 |
| 7. | Cetyl ethylhexanoate | 1.2 |
| 8. | KF-7312L (Note 5) | 2 |
| 9. | KSP-300 (Note 6) | 2 |
| 10. | KF-56A (Note 7) | 3 |
| 11. | KF-4418 (Note 8) | 3 |
| 12. | Dispersion of any of Examples 31 to 44 | 20 |
| 13. | BG | 5 |
| 14. | Aqueous PCA-Na solution | 1 |
| 15. | Ethylhexylglycerin | q.s. |
| 16. | Pentylene glycol | 1 |
| 17. | Caprylyl glycol | q.s. |
| 18. | Sodium citrate | q.s. |
| 19. | Sodium chloride | 0.5 |
| 20. | Purified water | 41.4 |
| Total | | 100.0 |

| | | |
|---|---|---|
| (Note 1) Mixture of 70-80% dimethicone (6 cs) + 20-30% (dimethicone/polyglycerin-3) crosspolymer, Shin-Etsu Chemical Co., Ltd. (Note 2) Mixture of 65-75% triethylhexanoin + 25-35% (vinyl dimethicone/lauryl dimethicone) crosspolymer, Shin-Etsu Chemical Co., Ltd. (Note 3) Lauryl polyglyceryl-3 polydimethylsiloxyethyl dimethicone, Shin-Etsu Chemical Co., Ltd. (Note 4) Methyl trimethicone, Shin-Etsu Chemical Co., Ltd. (Note 5) 50%Trimethylsiloxysilicate solution, Shin-Etsu Chemical Co., Ltd. (Note 6) (Diphenyl dimethicone/vinyl diphenyl dimethicone/silsesquioxane) crosspolymer, Shin-Etsu Chemical Co., Ltd. (Note 7) Diphenylsiloxyphenyl trimethicone, Shin-Etsu Chemical Co., Ltd. (Note 8) Caprylyl methicone, Shin-Etsu Chemical Co., Ltd. | | |

### (Production method)

A: Ingredients 1 to 11 were mixed uniformly.
B: Ingredients 13 to 20 were mixed uniformly.
C: The mixture obtained in B was added to the mixture obtained in A and emulsified, and ingredient 12 was added thereto to give a W/O liquid foundation.

The W/O liquid foundation obtained, in spite of being highly loaded with powder, had good spreadability and a good feeling on use.

### [Example 46] W/O cream foundation

| Composition | | % |
|---|---|---|
| 1. | KSG-330 (Note 1) | 3 |
| 2. | KSG-18A (Note 2) | 5 |
| 3. | KF-6038 (Note 3) | 1 |
| 4. | KF-6048 (Note 4) | 2 |
| 5. | Organically modified clay mineral | 1 |
| 6. | Hexyl laurate | 5 |
| 7. | Homosalate | 3 |
| 8. | Bis-ethylhexyloxyphenol methoxyphenyl triazine | 2 |
| 9. | Ethylhexyl salicylate | 3 |
| 10. | KF-56A (Note 5) | 3 |
| 11. | KP-549 (Note 6) | 3 |
| 12. | Dispersion of any of Examples 31 to 44 | 15 |
| 13. | BG | 5 |
| 14. | Betaine | 1 |
| 15. | Dipotassium glycyrrhizinate | 0.1 |
| 16. | Methyl gluceth-10 | 1 |
| 17. | Caprylhydroxamic acid | q.s. |
| 18. | Sodium citrate | q.s. |
| 19. | Sodium chloride | 0.5 |
| 20. | Purified water | Balance |
| Total | | 100.0 |

| | | |
|---|---|---|
| (Note 1) Mixture of 75-85% triethylhexanoin + 15-25% (PEG-15/lauryl dimethicone) crosspolymer, Shin-Etsu Chemical Co., Ltd. (Note 2) Mixture of 80-90% diphenylsiloxyphenyl trimethicone + 10-20% (dimethicone/phenyl vinyl dimethicone) crosspolymer, Shin-Etsu Chemical Co., Ltd. (Note 3) Lauryl PEG-9 polydimethylsiloxyethyl dimethicone, Shin-Etsu Chemical Co., Ltd. (Note 4) Cetyl PEG/PPG-10/1 dimethicone, Shin-Etsu Chemical Co., Ltd. (Note 5) Diphenylsiloxyphenyl trimethicone, Shin-Etsu Chemical Co., Ltd. (Note 6) 40% (Acrylates/dimethicone) copolymer solution, Shin-Etsu Chemical Co., Ltd. | | |

### (Production method)

A: Ingredients 1 to 12 were mixed uniformly.
B: Ingredients 13 to 20 were mixed uniformly.
C: The mixture obtained in A was added to the mixture obtained in B and emulsified to give a W/O cream foundation.

The W/O cream foundation obtained, in spite of being highly loaded with powder, had good spreadability and a good feeling on use.

## Claims

1. An organopolysiloxane represented by formula (1) below: 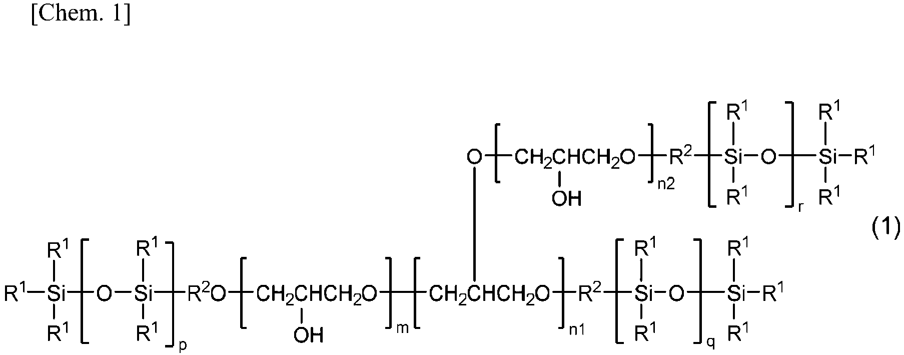 wherein R¹ independently at each occurrence is a group selected from C1-C20 alkyl groups, C6-C20 aryl groups, and C7-C20 aralkyl groups; R² independently at each occurrence is a C3-C15 divalent organic group optionally interrupted by an oxygen atom; p is an integer of 0 ≤ p ≤ 9; q is an integer of 0 ≤ q ≤ 200; r is an integer of 0 ≤ r ≤ 200; m is an integer of 0 ≤ m ≤ 10; n1 is an integer of 0 ≤ n1 ≤ 5 (with a proviso that when m = 0 or 1, 1 ≤ n1 ≤ 5); n2 is an integer of 0 ≤ n2 ≤ 5; and the glycerin units in the brackets followed by m and n1 are each optionally bonded to one another blockwise or randomly.

2. The organopolysiloxane according to claim 1, wherein in formula (1), n1 = 0 and m is 0 < m ≤ 10.

3. The organopolysiloxane according to claim 2, wherein in formula (1), p ≠ q.

4. The organopolysiloxane according to claim 1, wherein the organopolysiloxane has a polystyrene-equivalent weight average molecular weight of 1,000 to 20,000.

5. The organopolysiloxane according to claim 1, wherein in formula (1), q is 0 ≤ q ≤ 9 and r is 0 ≤ r ≤ 9.

6. The organopolysiloxane according to claim 1, wherein in formula (1), q is 10 ≤ q ≤ 200.

7. A cosmetic composition comprising the organopolysiloxane described in any one of claims 1 to 6.

8. A dispersion comprising the organopolysiloxane described in any one of claims 1 to 6, a powder, and an oil.

9. The dispersion according to claim 8, wherein the organopolysiloxane is represented by formula (1) in which q is 0 ≤ q ≤ 9 and r is 0 ≤ r ≤ 9, and the powder has an average primary particle size of less than 15 nm.

10. The dispersion according to claim 8, wherein the organopolysiloxane is represented by formula (1) in which 10 ≤ q ≤ 200, and the powder has an average primary particle size of 15 nm or more.

11. A cosmetic composition comprising the dispersion described in claim 8.
